(19) Europäisches Patentamt
European Patent Office
Office européen des brevets

(11) **EP 2 394 694 A1**

(12) **EUROPÄISCHE PATENTANMELDUNG**

(43) Veröffentlichungstag:
**14.12.2011 Patentblatt 2011/50**

(51) Int Cl.:
*A61N 1/05* (2006.01)    *A61N 1/44* (2006.01)

(21) Anmeldenummer: **11004753.7**

(22) Anmeldetag: **10.06.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **10.06.2010  EP 10005994**

(71) Anmelder:
• **OOO "Novye Energeticheskie Technologii"**
  **Moskau 107045 (RU)**
• **Golsen Limited**
  **3041 Limassol (CY)**

(72) Erfinder:
• **Frolov, Sergey A.**
  **117042 Moskow (RU)**
• **Sushkov, Oleg I.**
  **107150 Moskow (RU)**

• **Pshelenskaya, Anna I.**
  **601911 Kovrov (RU)**
• **Belov, Sergey V.**
  **119311 Moskow (RU)**
• **Danileyko, Yuri K.**
  **142092 Troitsk (RU)**
• **Nefedov, Sergey M.**
  **115612 Moskow (RU)**
• **Osiko, Vjacheslav V.**
  **117420 Moskow (RU)**
• **Salyuk, Viktor A.**
  **117321 Moskow (RU)**

(74) Vertreter: **Jeck, Anton**
  **Jeck Fleck Herrmann**
  **Patentanwälte**
  **Postfach 14 69**
  **71657 Vaihingen/Enz (DE)**

(54) **Elektrochirurgische Einrichtung zur Behandlung von Entzündungen mittels invasiver Elektrostimulation**

(57)    Die Erfindung betrifft eine elektrochirurgische Einrichtung zur Behandlung von Entzündungen mittels invasiver Elektrostimulation mit einer aktiven Elektrode und einer passiven Elektrode, wobei die aktive Elektrode in einem Applikator eingebettet ist und einen Innenkanal für die Zuführung einer leitfähigen Flüssigkeit (A) in das Behandlungsgebiet (C) eines biologischen Gewebes sowie einen Isoliermantel auf seiner Außenoberfläche über die Länge ihres distalen Teils bis auf das offene Ende des distalen Teils sowie ein mit der aktiven Elektrode verbundenes System zur HF-Stromversorgung aufweist, wobei der HF-Strom die Erzeugung eines Niedertemperaturplasmas am Ende des distalen Teils der aktiven Elektrode in Anwesenheit der leitfähigen Flüssigkeit (A) sicherstellt und wobei die passive Elektrode an den Körper des Patienten anlegbar ist. Eine maximale Automatisierung des Betriebs der Einrichtung wird dabei dadurch erreicht, dass für die einzelnen Schritte des Bearbeitungsablaufs eigene Einrichtungen vorgesehen sind, die zugeordnete Funktionen automatisch ausführen, überwachen und kontrollieren.

Fig. 1

EP 2 394 694 A1

**Beschreibung**

Die Erfindung betrifft eine elektrochirurgische Einrichtung zur Behandlung von Entzündungen mittels invasiver Elektrostimulation nach dem Oberbegriff des Anspruchs 1.

Die Erfindung ist in der Medizin und zwar in der Chirurgie und Dermatologie einsetzbar. Sie kann zur Behandlung von verschiedenen Entzündungsformen, darunter chronischen Entzündungen, anhand der Stimulation von Kupiervorgängen und der Behandlung von Entzündungen unter Einwirkung von HF-Stromimpulsen mit teilweiser Modifikation der Struktur des biologischen Gewebes im Herd oder rings um den Entzündungsherd herum eingesetzt werden. Eine solche Stimulation führt zur Aktivierung des Immunsystems im Lokalisationsbereich des Entzündungsherds. Bekanntlich ist das Entstehen von akuten Entzündungsherden und die Verbreitung von chronischen Entzündungen durch eine Änderung der Pathogenität der Mikroflora, durch eine Zunahme der Endokrinerkrankungsrate und folglich durch einen Abbau der Immunabwehr des Körpers begünstigt. Das trägt zur Umwandlung der Erkrankung in chronische Formen bei.

Aus dem Stand der Technik sind verschiedene chirurgische und therapeutische Verfahren zur Behandlung von Entzündungskrankheiten, darunter von akuten Entzündungen, bekannt.

Die chirurgischen Behandlungsmethoden beruhen auf einer chirurgischem Einwirkung mit der Öffnung und der Drainage des Entzündungsherds und mit einer nachfolgenden Bearbeitung mit bakteriziden Mitteln (B. T. Glukhen'ky, V. V. Delektorsky, R. F. Fyodorovskaya. Pustulöse Hauterkrankungen, Kiev, 1983). Solche Verfahren sind schmerzhaft, traumatisch und verlangen eine lange Rehabilitation. Zudem verursachen sie die Bildung von grobfaserigem Narbengewebe. Ein solches Narbengewebe ersetzt das funktionsaktive parenchymatöse Organgewebe. Dies kann im Endeffekt zu einer funktionellen Minderwertigkeit des Organs führen. So wird z. B. bei der Öffnung des pathologischen Herds nach dem elektrischen Dissektionsverfahren (Marc R.Avram, Sandy Tsao, et all. Color Atlas of Cosmetic Dermatology, Mc Grow Hill Medical, New York, 2006, p. 295) eine weite Zone einer thermischen Destruktion der umliegenden Gewebe an der Durchtrennungsstelle gebildet. Die thermisch koagulierten Gewebe erweitern den Schadensbereich. Dies führt zu einer andauernden Rehabilitationsphase und zur Bildung eines grobfaserigen, das parenchymatöse Organgewebe ersetzenden Bindegewebes.

Die bekannten therapeutischen Verfahren zur Behandlung schließen eine Einwirkung auf die pathologischen Gewebe und die pathologische Mikroflora mit Hilfe von Antibiotika-Therapie und medikamentöser Korrektur des Immunsystems ein (A. V. Shapiro. Antibiotika und ihre Anwendung bei eitrigen Entzündungen, M., 1999). Jedoch stellen die therapeutischen Behandlungsmethoden in der Regel nur ein Kupieren der akuten Entzündung sicher, indem die unterakute Entzündung in eine chronische Form versetzt wird. Die Behandlung zieht sich in die Länge und setzt die Anwendung von radikaleren Behandlungsmethoden voraus, z. B. eines wiederholten chirurgischen Eingriffs.

Aus dem Stand der Technik sind verschiedene Einwirkungsverfahren bekannt, um die Aktivität des Immunsystems des Körpers bei der Behandlung von akuten Entzündungen zu steigern:

- eine medikamentöse Therapie, welche auf einer chemischen Einwirkung auf die intrazellulären Vorgänge beruht;
- eine physische Therapie, bei der zu Behandlungszwecken physische Faktoren angewendet werden, wobei diese Faktoren durch Transformation von bestimmten Energiearten in die Energie der biologischen Vorgänge erzeugt werden. So sind z. B. folgende Verfahren bekannt:

    - ein Verfahren, bei welchem künstliche Lichtstrahlungen, z. B. Laserstrahlung (infrarot über UV bis monochromatische kohärente Strahlung) verwendet werden;
    - die Elektrotherapie und Franklinisation (statische Elektrizität);
    - eine Anwendung von Galvanisation und Arzneimittelelektrophorese (galvanischer Strom);
    - die Faradotherapie (asymmetrischer Wechselstrom);
    - die Tesla-Ströme-Behandlung (Arsonvalisation) oder Diathermie (HF-Ströme);
    - Induktothermie, UHF-Therapie, Höchstfrequenztherapie (magnetische, elektrische und elektromagnetische Felder der Nieder-, Hoch-, Ultrahoch- und Höchstfrequenzen), mechanische Schwingungen (Infraschall- bis Ultraschallschwingungen).

Bekanntlich ruft die Anwendung von physisch-therapeutischen Einwirkungsmethoden sowohl unspezifische als auch spezifische Antwortreaktionen des Körpers hervor. Sie sind durch die Besonderheiten des Wirkungsträgers und des pathologischen Prozesses bedingt und stellen den Haupttheileffekt - die Aktivitätssteigerung des Immunsystems des Körpers - sicher. Um den größten therapeutischen Effekt bei minimaler Belastung für den Organismus mittels Verstärkung der spezifischen und Abschwächung der unspezifischen Wirkungskomponente zu erreichen, werden die physischen Faktoren öfters im Intervallbetrieb (impulsbetrieb) und in kleinen Dosen angewendet. Die Wahl des Wirkungsfaktors, seiner Dosis und der Anwendungsweise sowie der Kombination von einigen Faktoren werden durch die Form und das Krankheitsstadium und den Zustand des Organismus bestimmt. Die physisch-therapeutischen Einwirkverfahren sind in der Anfangsphase der Krankheit bei funktionellen Störungen wirksam. Allerdings werden sie auch bei anderen Behandlungsphasen und im Rahmen der medizinischen Rehabilitation angewendet, um bestimmte Vorgänge im Körper auf verschiedenen Ebenen einschließlich der zellularen und der molekularen Ebene zu beeinflussen.

Aus der RU 2202382, C2 ist ein Verfahren zur Innengewebe-Elektrostimulation von vegetativen Nervenbildungen bei der Behandlung von Bronchialasthma und obstruktiver Bronchitis bekannt. Die Elektrostimulation erfolgt mittels der Beanspruchung des sympathischen Nervensystemabschnitts mit einem Impulsstrom von 5 - 20 mA mit einer Frequenz von 10 - 25 Hz bei einer Impulsdauer von 0,5 ms der Kathode. Die Einwirkung dauert 20 Minuten. Damit ist die Korrektur von Immunstörungen des Körpers und zwar die Normalisierung der Menge und die Funktionsaktivität der T-Lymphozyte, die Beseitigung von Imbilanz von Unterpopulationen und die Aktivierung von Phagozytose sichergestellt. Dabei ist als Kathode eine mit der Impulsstromquelle verbundene sterile Injektionskanüle verwendet. Der Chirurg taucht die Injektionskanüle in den Einwirkungsbereich in der gewünschten Tiefe ein. Dabei lässt er sich von seinen Fachkenntnissen und Fertigkeiten leiten.

Es ist das Problem der Behandlung von unterschiedlichen akuten und chronischen kutanen und subkutanen Entzündungsformen bekannt. Sie sind durch die eitererregenden Mikroorganismen, z. B. Pyodermien, hervorgerufen. Es handelt sich dabei um eine Oberflächen-Staphylodermie (Ostiofolliculitis, Folliculitis superficialis, Sycosis vulgaris, Hautfinnen, pemphigus neonatorum epidemicus) und eine tiefe Staphylodermie (tiefe Follikulitis, Furunkel, Furunkulose, Karbunkel, Hydradenitis), eine Oberflächen-Streptodermie (impetigo streptogenes einschließlich der angulus infectiosus streptococcicus, impetigo impetiginosum, impetigo bullosa, impetigo circinata, impetigo syphiloides, panaritium superficiale, pityriasis simplex) und eine tiefe Streptodermie (Pannikulose einschließlich akuter Pannikulose, cellulitis streptogenes erysipelas, ecthyma vulgare) oder eine Oberflächen-Strepto-Staphylodermia (impetigo vulgaris) und eine tiefe Strepto-Staphylodermia (pyodermia chronica ulcerosa, pyodermia chancriformis). Bei solchen Erkrankungen ist die langsame und andauernde Erwärmung des infizierten biologischen Gewebes im Entzündungsherd kontraindiziert.

Aus der Patentschrift RU 2383365, C1 ist ein Verfahren zur Behandlung von verschiedenen akuten und chronischen Formen von Pyodermie anhand einer invasiven Elektropunktur bekannt. Dabei wird ein Periode-Impuls-Betrieb der Stromgebung in einer Funkfrequenzbandbreite mit einer Impulsdauer von 200 $\pm$ 50 $\mu$Sek. und mit einer Impulsfolgefrequenz von 1000 $\pm$ 100 Hz angewendet. Der Strom wird an eine nadelförmige Elektrode mit einem Durchmesser von 0,3 - 0,5 mm angelegt. Die Elektrode wird mit einer Tiefe von 3 - 12 mm eingebracht. Gemäß diesem Verfahren wird das Operationsfeld des biologischen Objekts mit einem Gewebe-Antiseptikum versorgt. Die inaktive Elektrode wird an den Körper des Patienten (biologisches Objekt) angelegt. Danach wird die nadelförmige aktive (differente) Elektrode in das biologische Gewebe eingeführt. Der HF-Strom wird eingeschaltet. Die Energieabgabe pro Längeneinheit der Nadel muss 5 - 7 J/cm innerhalb einer Zeitspanne von 10 Sek. betragen, da bei einer solchen Energiedichte der beste klinische Effekt zu beobachten ist. Die Expositionswahl ist durch die anatomischen und physiologischen Hautbesonderheiten festgelegt. Nach dem Einschalten des HF-Stroms wird die Nadelelektrode aus dem biologischen Gewebe herausgezogen. Die Nadeleinführung in die Haut des biologischen Objekts wird wie folgt durchgeführt: bei Herd-Pyodermien am Umfang des Entzündungsherds, bei verbreiteten Pyodermiearten gemäß den Hautlinien mit einem Schritt von 8 $\pm$ 2 mm zwischen den benachbarten Durchstichen und zwischen den Reihen.

Für die Ausführung des Verfahrens ist eine Einrichtung mit einer Stromversorgung, einer aktiven und wenigstens einer inaktiven (inversen) Elektrode verwendet. Die Stromversorgung stellt den Periode-Impuls-Betrieb der Stromgebung in der Funkfrequenzbandbreite mit einer Impulsdauer von 200 $\pm$ 50 $\mu$Sek und einer Impulsfolgefrequenz von 1000 $\pm$ 100 Hz sicher. Die aktive (differente) Elektrode berührt dabei über ihre gesamte Länge das biologische Gewebe. Deswegen kann die Überschreitung der Energieabgabe bei diesem Verfahren zu einer Koagulation des biologischen Gewebes und zu Brandverletzungen führen. Das setzt einen längeren Vorgang der Ausheilung des Elektropunkturgebiets voraus. Dabei muss die aktive (differente) nadelförmige Elektrode hochsteril sein, um eine Nachinfektion der gebildeten Wunde auszuschließen. Das ist im Falle eitererregender und sporenbildender Mikroorganismen problematisch. Die Besonderheiten der Wahl der Betriebsarten und der Dauer der Elektroimpulseinwirkung innerhalb der genannten Bandbreiten sowie die Besonderheiten des Hantierens mit der Nadel, nämlich die Wahl und das Erreichen der erforderlichen Tiefe der Elektropunktur, sind durch die Fachkenntnisse und die Fertigkeiten des Chirurgen festgelegt.

Aus dem Patent US 2003/0028189, A1 ist ein System zur elektrochirurgischen Behandlung eines biologischen Gewebes mittels einer Beanspruchung mit Hochfrequenzspannung in Anwesenheit einer leitfähigen Flüssigkeit bekannt, um Niedertemperaturplasma für die Ablation des biologischen Gewebes im Plasma oder beim Kontakt mit Plasma zu bilden. Dieses System enthält eine elektrochirurgische Sonde oder einen Katheter, welche(r) mit einem Isoliermantel über die gesamte Länge bis auf das distale Ende versehen ist. Die Sonde bzw. der Katheter sind in diesem System dafür angepasst, um im Behandlungsgebiet so angeordnet zu werden, dass eine oder mehrere aktive Elektroden das biologische Gewebe kontaktieren oder in der Nähe des zu behandelnden biologischen Gewebes in Anwesenheit der leitfähigen Flüssigkeit positioniert werden. Die leitfähige Flüssigkeit wird dem Behandlungsgebiet durch ein Loch in der aktiven Elektrode - der Sonde oder des Katheters - zugeführt. Nach der Positionierung der Elektrode wird zwischen der aktiven Elektrode und einer oder mehreren inversen Elektroden eine Hochfrequenzspannung erzeugt. Damit werden eine nichtthermische Plasmabildung rings um die aktive Elektrode und die Volumenentfernung oder Ablation von wenigstens einem Stück des biologischen Gewebes gesichert. Die Hochfrequenzspannung erzeugt elektrische Felder rings um die aktiven Elektroden. Die Energie dieser Felder ist für eine Ionisation der leitfähigen Flüssigkeit im Anordnungsbereich der aktiven Elektroden erzeugt. In der Menge des ionisierten Gases oder Plasmas werden freie Elektronen gebildet. Die Elektronen- und Atomstöße verursachen eine Freisetzung einer großen Menge von Elektronen. Dieser Vorgang hat einen Stufencharakter, solange das Plasma eine ausreichende Energiemenge aufweist, die für die Zerstörung der Moleküle des biologischen Gewebes erforderlich ist. Eine solche Zerstörung ist durch eine Moleküldissoziation und Ablation hervorgerufen. Dabei liegt die Temperatur des gebildeten Plasmas unter 100° C und in der Regel sogar unter 80° C. Bei den elektrischen Impulsfrequenzen von 50 kHz bis 20 MHz muss dies Brandverletzungen und die Koagulation des biologischen Gewebes ausschließen. Während der Behandlung wird die Plasmatemperatur mittels Geber überwacht. Die leitfähige Flüssigkeit wird als Elektrolyt verwendet und ist gleichzeitig die plasmabildende Lösung.

Das oben beschriebene System stellt eine elektrochirurgische Bearbeitung des biologischen Gewebes zwecks Ablation oder Entfernung von Massen des biologischen Gewebes infolge einer Zersetzung des biologischen Gewebes in die einfachsten Verbindungen mittels einer Loslösung der Molekülbindungen sicher. Eine solche Loslösung erfolgt unter der Wirkung von Plasma bei einem vorliegenden tatsächlichen Kontakt zwischen dem Plasma und dem zu entfernenden oder zur Ablation vorgesehenen biologischen Gewebe. Der unmittelbare Kontakt zwischen dem Plasma und dem biologischen Gewebe kann eine Hitzeverbrennung und Koagulation des biologischen Gewebes, insbesondere im Bereich zwischen der aktiven und der passiven Elektrode, verursachen. Deswegen werden für die genannten Zwecke Hochfrequenzbetriebsarten je nach den Bedingungen der durchzuführenden Operation, den Strukturbesonderheiten des biologischen Gewebes und je nach der gegenseitigen Anordnung der Elektroden gewählt. Bei einer solchen elektrochirurgischen Bearbeitung entstehen Wundflächen. Diese bedürfen einer nachfolgenden therapeutischen Behandlung mit mehrmaliger Versorgung der Wunde mit bakteriziden Mitteln, der Einnahme von Antibiotika und einer Stimulation des Immunsystems des Körpers, um die Ausheilung der Wunden zu erreichen.

Es ist Aufgabe der Erfindung, eine elektrochirurgische Einrichtung zur Behandlung von Entzündungen zu entwickeln. Diese Einrichtung soll eine hohe Effizienz des Kupierens haben und die Behandlung von Entzündungen des biologischen Gewebes sicherstellen. Die Entzündungen können unter anderem durch eine Infizierung mit eitererregenden und sporenbildenden Mikroorganismen hervorgerufen sein. In der Einrichtung sind invasive Verfahren der Stimulation des Immunsystems angewendet, bei denen der Strom den den Entzündungsherd umgebenden Geweben oder unmittelbar dem Entzündungsherd zugeführt wird. Die Stromzuführung erfolgt im Periode-Impuls-Betrieb bei niedrigen Temperaturen, ohne dass sich offene Wundflächen bilden. Dabei sind eine Infizierungsgefahr der bei der Einführung der Elektrode in das Behandlungsgebiet verletzten Bereiche sowie die Gefahr einer Hitzeverbrennung und Koagulation des biologischen Gewebes vermieden. Dabei sollen auch die Kontrolle der Wahl und die Einhaltung von den dem Zustand der Entzündungsherde entsprechenden Behandlungsarten sichergestellt sein.

Die entwickelte elektrochirurgische Einrichtung zur invasiven Elektroimpulsbearbeitung des biologischen Gewebes im Entzündungsgebiet und um das Entzündungsgebiet herum erfolgt unter Anwendung von Niedertemperaturplasma einer Glimmentladung im Gasmedium. Das Gasmedium ist infolge der Verdampfung einer leitfähigen Flüssigkeit gebildet. Die leitfähige Flüssigkeit befindet sich dabei im Pufferbereich zwischen dem Plasma und dem biologischen Gewebe. Diese leitfähige Flüssigkeit ist durch einen Hochfrequenzstrom bis zu einer Temperatur erwärmt, bei der eine Hitzeverbrennung und eine Koagulation des mit der leitfähigen Flüssigkeit kontaktierenden biologischen Gewebes ausgeschlossen sind. Diese Temperatur stellt dabei eine Ionisation der Dämpfe der leitfähigen Flüssigkeit sicher. Dabei ist in dem mit dem biologischen Gewebe kontaktierenden Pufferbereich eine bakterizide Lösung gebildet. Die bakterizide Lösung sorgt für eine Destruktion der Molekularstruktur der oberflächennahen Schichten des biologischen Gewebes im Behandlungsgebiet, um eine Immunantwort zu erhöhen. Die Immunantwort führt zu einer Aktivierung des Immunsystems im Bereich des Entzündungsherds. Dabei ist die Aufgabe gestellt, die Kontrolle und die Regelung des Vorgangs der invasiven Elektroimpulsbehandlung und einer bakteriziden Behandlung des biologischen Gewebes sicherzustellen, um eine maximale Automatisierung unter solchen Bedingungen zu erreichen, bei denen keine visuelle Kontrolle der Behandlungsvorgänge bei subkutaner Anordnung der Behandlungsbereiche des biologischen Gewebes möglich ist.

Die gestellte Aufgabe ist durch die Merkmale des Anspruchs 1 gelöst. Die Einrichtung weist auf:

-    eine aktive (differente) Elektrode, die in einen Applikator eingebaut ist und folgende Merkmale aufweist:

     -    einen Innenkanal für die Zuführung der leitfähigen Flüssigkeit in das Behandlungsgebiet des biologischen Gewebes;
     -    einen Isoliermantel auf der Außenoberfläche der Elektrode über die Länge ihres distalen Teils bis zum offenen Ende des distalen Teils und
     -    ein mit der aktiven Elektrode verbundenes System zur HF-Stromversorgung, wobei dieser Hochfrequenzstrom die Erzeugung eines Niedertemperaturplasmas am Ende des distalen Teils der aktiven Elektrode in Anwesenheit der leitfähigen Flüssigkeit sicherstellt,

     und

- eine passive Elektrode, die am Körper des Patienten anbringbar ist.
  Nach der Erfindung ist vorgesehen,
- dass sie für die Durchführung von Elektrostimulation in der Nähe des Entzündungsherds mittels Destruktion der Molekularstruktur der oberflächennahen Schichten des biologischen Gewebes und gleichzeitiger bakterizider Behandlung des biologischen Gewebes beim Kontakt des biologischen Gewebes mit einem leitfähigen Pufferbereich angepasst ist, wobei der Pufferbereich das Plasma umringt und rings um das distale Ende der aktiven Elektrode ausgebildet ist,
- dass die aktive Elektrode dabei eine Spitze aufweist und in das biologische Gewebe vorher einbringbar ist, wobei die Eintauchtiefe die Tiefe des Behandlungsgebiets mit der leitfähigen Flüssigkeit überschreitet,
- dass die leitfähige Flüssigkeit im Voraus und zwar beim Eintauchen des distalen Endes in das Behandlungsgebiet aus einem Behälter zuführbar ist, welcher am proximalen Ende der aktiven Elektrode angeordnet ist,
- dass die Einrichtung dabei die Durchführung der genannten Behandlung bei der Austauschung (Entnahme) der Elektrode aus dem biologischen Gewebe sicherstellt und Folgendes aufweist:

  - einen Applikator, der das Austauschen der aktiven Elektrode nach draußen mit vorgegebener Geschwindigkeit sicherstellt, wobei das Austauschen über die Stirnfläche des Applikators um einen Abstand erfolgt, welcher seiner vorgegebenen Eintauchtiefe im biologischen Gewebe gleich ist, dass dabei der Berührungszeitpunkt zwischen der aktiven Elektrode und der Körperoberfläche des Patienten erfasst ist und dieser Zeitpunkt als Anhaltspunkt für die Bestimmung der Eintauchtiefe dient und dass das Austauschen (die Entnahme) der Elektrode aus dem biologischen Gewebe mit einer vorgegebenen Geschwindigkeit sichergestellt ist, wobei diese Geschwindigkeit die vorgegebene Behandlungsdauer des biologischen Gewebes innerhalb der Austauschstrecke des Elektrodeendstücks und zwar entlang der Behandlungsgebietstiefe bis zur Grundstellung der Elektrode sichert,

- dass ein System für die Zuführung der leitfähigen Flüssigkeit vorgesehen ist, das für die Zuführung einer vorgegebenen Menge der leitfähigen Flüssigkeit über einen Behälter und einen Innenkanal der aktiven Elektrode mit einer vorgegebenen Geschwindigkeit innerhalb der Einführungsdauer der aktiven Elektrode in das biologische Gewebe sorgt, dass die genannte vorgegebene Geschwindigkeit erforderlich ist, um rings um den distalen Teil der aktiven Elektrode bei ihrer Eintauchung in das biologische Gewebe einen Pufferbereich zu erzeugen, dass dabei der Pufferbereich den unmittelbaren Kontakt zwischen dem Plasma und dem biologischen Gewebe verhindert und vorgegebene Maße in Übereinstimmung mit dem Umfang des distalen Endes der Elektrode aufweist, die in das biologische Gewebe in der Tiefe des Behandlungsgebiets eingetaucht ist,
- dass ein HF-Stromversorgungssystem vorgesehen ist, welches eine HF-Stromversorgung einer leitfähigen Flüssigkeit innerhalb der Zeitspanne des Austauschens der Elektrode aus dem Behandlungsgebiet sicherstellt, dass das HF-Stromversorgungssystem im Innenraum der aktiven Elektrode eingerichtet ist, dass die HF-Stromversorgung in einem Periode-Impuls-Betrieb mit solchen Kennwerten erfolgt, welche die Erzeugung eines Niedertemperatur-Plasmas mit einer Glimmentladung innerhalb des Pufferbereichs am Ende des unisolierten Distalteils der aktiven Elektrode bewerkstelligt und die Produktion einer vorgegebenen Menge von Wasserstoffperoxid im Pufferbereich sicherstellt, dass diese Menge von Wasserstoffperoxid für eine Stimulation und eine bakterizide Behandlung des biologischen Gewebes im Behandlungsgebiet ausreicht,
- dass eine automatische Steuerungseinheit mit einem System zum Empfang und zur Übertragung von elektrischen Signalen von und zu den genannten Funktionssystemen der Einrichtung, einem Mikrorechner, einer Fußtaste zur Fernein- und - ausschaltung der Einrichtung, einer Stromversorgung, einem Steuerschalter vorgesehen ist, dass die automatische Steuerungseinheit zum Ein- und Ausschalten der Funktionssysteme der Einrichtung sowie für eine Aktualisierung ihrer Betriebszustände sorgt und folgende Funktionen erfüllt:

  - eine Auswahl der Betriebsparameter der Einrichtung je nach den durch den Chirurgen gewählten Kriterien gemäß dem Charakter des biologischen Gewebes im Behandlungsgebiet, nach dem Zustand des Entzündungsherds, nach den Maßen des distalen Endes der aktiven Elektrode, nach ihrer Eintauchtiefe im biologischen Gewebe und je nach der Tiefe des Behandlungsbereichs des biologischen Gewebes;
  - eine Bildung und Online-Aktualisierung (Aufrechterhaltung des aktiven Zustands) der Datenbanken der Parameter der genannten Systeme der Einrichtung, welche für die Erzeugung von Steuerbefehlen erforderlich sind, wobei diese Befehle das Ein- und/oder Ausschalten bzw. die Aktualisierung der Parameter oder die Unterbrechung bzw. Sperrung von einzelnen Operationen oder eine Not-Abschaltung der Einrichtung verursachen;
  - eine Weiterleitung der Steuerbefehle an die genannten Systeme der Einrichtung;
  - eine Überwachung der Einhaltung der genannten Parameter aufgrund der Auswertung der elektrischen Signale von den genannten Systemen der Einrichtung;
  - eine digitale Verarbeitung der empfangenen elektrischen Signale und

-	eine Visualisierung der Betriebsparameter der Systeme der Einrichtung anhand graphischer und/oder Textmittel und eine Anzeige des Einrichtungsbetriebs.

Dabei weist der Applikator gemäß der Erfindung zweckmäßig Folgendes auf:

-	ein Gehäuse aus einem Nichtleiterwerkstoff zur Aufnahme der aktiven Elektrode, wobei das Gehäuse mit einem leitfähigen Kontaktflansch für die Platzierung des Applikators am Körper des Patienten versehen ist und ein Mittenloch für eine lockere Aufnahme des distalen Teils der aktiven Elektrode aufweist, wenn die Elektrode aus dem Gehäuse des Applikators in die Arbeitsstellung herausgezogen ist, um ihre Eintauchtiefe in das biologische Gewebe einzunehmen,
-	einen Berührungssensor zur Signalisierung der Berührung zwischen der Spitze der genannten aktiven Elektrode und der Körperoberfläche des Patienten, wobei dieser Berührungssensor für die Erfassung des Stroms sorgt, welcher zwischen dem Kontaktflansch und der aktiven Elektrode auftritt, wenn diese die Körperoberfläche des Patienten berühren, und
-	ein Antriebssystem der aktiven Elektrode mit einem Weggeber der aktiven Elektrode.

Dabei weist das Antriebssystem der aktiven Elektrode gemäß der Erfindung zweckmäßig Folgendes auf:

-	eine Zugstange, die über einen elektrischen Isolierkörper mit der Stirnseite des proximalen Endes der aktiven Elektrode und mit einem Linear-Schrittmotor verbunden ist, und
-	einen Weggeber der aktiven Elektrode, der auf der Zugstange angeordnet ist, wobei der Weggeber auf der Strecke von der Ausgangsstellung, (wenn die Spitze der genannten aktiven Elektrode die Körperoberfläche des Patienten berührt) bis er die vorgegebene maximale Eintauchtiefe im biologischen Gewebe erreicht hat, funktioniert.

Dabei weist das Antriebssystem der aktiven Elektrode gemäß der Erfindung zweckmäßig Folgendes auf:

-	einen Druckluftspeicher,
-	ein Getriebe,
-	einen elektrisch gesteuerten Verteiler mit einem steuerbaren Ventil,
-	einen Druckluftzylinder mit Kolben, wobei der Kolben mittels einer Stange und über einen elektrischen Isolierkörper mit der Stirnseite des proximalen Endes der aktiven Elektrode verbunden ist, und
-	einen an der Kolbenstange angeordneten Weggeber der aktiven Elektrode, wobei der Weggeber auf der Strecke von der Ausgangsstellung, (wenn die Spitze der genannten aktiven Elektrode die Körperoberfläche des Patienten berührt) bis die vorgegebene maximale Eintauchtiefe im biologischen Gewebe erreicht ist, funktioniert.

Dabei weist das System für die Zuführung der leitfähigen Flüssigkeit gemäß der Erfindung zweckmäßig Folgendes auf:

-	einen Behälter für die leitfähige Flüssigkeit unter Druck,
-	eine Pumpe,
-	einen Druckgeber,
-	ein elektrisch gesteuertes Ventil und
-	eine Leitung für die Zuführung der leitfähigen Flüssigkeit zum Innenraum der aktiven Elektrode.

Dabei weist das HF-Stromversorgungssystem gemäß der Erfindung zweckmäßig Folgendes auf:

-	einen Hochfrequenz-Impulsgenerator,
-	einen Stromgeber,
-	einen Integrator zur Berechnung der Strommenge des durch das Plasma fließenden Stroms,
-	einen Stromoberwellenanalysator zur Kontrolle des Vorhandenseins der dritten Oberwelle und der Fundamentalschwingung, welche das Entstehen der Plasmaentladung kennzeichnen,
-	ein Analysegerät des optischen Plasmastrahlungsspektrums, wobei dieses Analysegerät zur Kontrolle der Plasmagastemperatur und des Plasmabetriebs dient. Für die Analyse ist das Intensitätsverhältnis der Linien der angeregten Natrium- oder Wasserstoffatome im Strahlungsspektrum benutzt. Das Analysegerät hat einen Lichtleiter. Ein Ende des Lichtleiters sitzt im proximalen Ende der aktiven Elektrode in der Nähe seines Innenkanals.

Dabei weist die Einrichtung gemäß der Erfindung zweckmäßig als Hochfrequenz-Impulsgenerator einen HF-Generator auf, welcher die Stromerzeugung mit einer Hauptträgerfrequenz im Frequenzbereich von 0,10 MHz bis 20,00 MHz mit der Möglichkeit einer Modulationsparameterregelung sicherstellt, die

- ein Impulspausenverhältnis im Bereich von 1 bis 10,
- eine Impulsfolgefrequenz im Bereich von 1 kHz bis 20 kHz und
- eine Spannungsimpulshöhe bei fehlender Belastung im Bereich von 100 V bis 400 V hat.

Dabei ist es gemäß der Erfindung zweckmäßig, dass das Analysegerät des optischen Plasmastrahlungsspektrums einen V-Verzweiger und lichtempfindliche Sensoren aufweist. Der V-Verzweiger ist optisch mit dem genannten Lichtleiter verbunden und sorgt für eine Trennung der Lichtemission des Plasmas in zwei optische Kanäle. Ein lichtempfindlicher Sensor ist mit dem Lichtfilter zur Aussonderung der Na-Linie von 337,1 nm oder der Wasserstoffatomlinie $H_\beta$ von 486,1 nm versehen. Ein weiterer lichtempfindlicher Sensor ist mit einem Lichtfilter zur Aussonderung der Na-Linie von 588,9 nm oder der Wasserstofflinie $H_\alpha$ von 656,3 nm versehen.

Außerdem ist es gemäß der Erfindung zweckmäßig, dass der Mikrorechner der automatischen Steuerungseinheit für die Erstellung und die zugriffsbereite Speicherung von wenigstens der folgenden Werte angepasst ist:

- die Intensität des Stroms, welcher die Funktion des Stromgebungssystems im Plasma-Erzeugungsbetrieb je nach Kennwerten der Impulseinwirkung sicherstellt,
- die Plasmatemperatur je nach dem Verhältnis der Strahlenintensitäten von Na- und Wasserstofflinien beim Übergang auf höhere Energieebenen,
- den Gesamtwert der durch das Plasma durchlaufenden Ladung,
- die Elektrodenverfahrgeschwindigkeit je nach der Größe des durch das Plasma fließenden Stroms,
- die Menge der leitfähigen Flüssigkeit, welche für die Bildung des Pufferbereichs je nach Durchmesser der aktiven Elektrode, ihrer Eintauchtiefe und der Tiefe des Behandlungsgebiets erforderlich ist,
- die Zuführungsgeschwindigkeit der leitfähigen Flüssigkeit je nach der Menge der leitfähigen Flüssigkeit im Pufferbereich und
- die Bewegungsgeschwindigkeit der Elektrode.

Dabei ist es gemäß der Erfindung zweckmäßig, dass der Mikrorechner für die Erstellung von Datenbanken der Geschwindigkeit $C_{equtv}$ der Erzeugung von Wasserstoffperoxid im Pufferbereich je nach der elektrischen Strommenge q, welche durch das Plasma gelaufen ist, angepasst ist. Die Geschwindigkeit $C_{equtv}$ der Wasserstoffperoxiderzeugung wird auf folgende Weise ermittelt:

$$C_{equtv} = F\left[1 - \exp\left(-\frac{G_0 kq}{V}\right)\right] / k$$

,

wobei

q für die Elektrizitätsmenge in Faraday,
$G_0$ für die Menge von Grammäquivalenten von Wasserstoffperoxid, welche ursprünglich pro Faraday der durchgelaufenen Elektrizität (Anfangsstromausbeute) gebildet werden,
V für die Menge der leitfähigen Flüssigkeit im Pufferbereich,
k für die Geschwindigkeitszahl der Wasserstoffperoxid-Zersetzungsreaktion und
F für die Erfahrungszahl, welche von den Kennwerten der leitfähigen Flüssigkeit abhängt,

stehen.

Dabei ist es gemäß der Erfindung zweckmäßig, dass als leitfähige Flüssigkeit eine 0,9%ige physiologische Kochsalzlösung eingesetzt ist.

Nachfolgend wird die Erfindung anhand von Ausführungsbeispielen einer elektrochirurgischen Einrichtung gemäß der Erfindung zur Behandlung von Entzündungen mittels invasiver Elektrostimulation sowie anhand der Beschreibung ihrer Anwendung anhand der beigefügten Zeichnungen näher erklärt. Es zeigen:

Fig. 1    das Schema einer elektrochirurgischen Einrichtung gemäß der Erfindung mit einem Ausbildungsbeispiel mit einem mechanischen Antrieb der aktiven Elektrode,

Fig. 2    das Schema einer elektrochirurgischen Einrichtung gemäß der Erfindung mit einem Ausbildungsbeispiel mit einem Druckluftantrieb der aktiven Elektrode und

Fig. 3    das Ablaufschema der Ausführung der Elektrostimulation und der bakteriziden Behandlung des biologischen Gewebes mit der elektrochirurgischen Einrichtung gemäß der Erfindung.

Die angeführten Ausbildungsbeispiele der Erfindung begrenzen dabei nicht ihre Ausgestaltungsmöglichkeiten, widersprechen nicht der technischen Idee, lassen eventuelle Verbesserungen und Weiterbildungen der Einrichtung zu und bleiben dabei innerhalb der Patentansprüche.

Die elektrochirurgische Einrichtung zur Behandlung von Entzündungskrankheiten mittels invasiver Elektrostimulation gemäß der Erfindung ermöglicht die Elektrostimulation in Kombination mit der bakteriziden Behandlung des biologischen Gewebes im Eintauchbereich der aktiven Elektrode im Behandlungsgebiet in einer Tiefe, die kleiner als die Eintauchtiefe ist, und im Bewegungsbereich des distalen Endes der aktiven Elektrode in der eingestellten Behandlungstiefe. Dabei stellt die Einrichtung Folgendes sicher:

- die Einführung der aktiven Elektrode in das Gebiet um den Entzündungsherd herum oder in den Entzündungsherd mit einer vorgegebenen Führungsgeschwindigkeit. Dabei wird um das distale Ende der aktiven Elektrode herum ein Pufferbereich aus leitfähiger Flüssigkeit erzeugt. Die leitfähige Flüssigkeit verhindert den Kontakt zwischen der Elektrode und der Oberfläche des biologischen Gewebes im Anstichbereich, indem eine erforderliche Menge der leitfähigen Flüssigkeit durch einen Raum innerhalb der aktiven Elektrode während der Einführung der Elektrode in das biologische Gewebe zugeführt wird. Dabei ist diese leitfähige Flüssigkeit gegenüber dem biologischen Gewebe biologisch passiv und gegenüber dem Blutplasma isotonisch,

- das Anlegen von HF-Strom in einem Periode-Impuls-Betrieb, um die Erzeugung von Niedertemperaturplasma mit einer Glimmentladung an der Spitze des distalen Teils der aktiven Elektrode zu starten. Die aktive Elektrode ist dabei im biologischen Gewebe um die Tiefe des Behandlungsgebiets in der Pufferbereich-Umgebung eingetaucht. Der Pufferbereich besteht aus der leitfähigen Flüssigkeit,

- das nachfolgende Austauschen (Ausziehen) der Elektrode aus dem Behandlungsgebiet gleichzeitig mit dem Anlegen des elektrischen HF-Stroms an die aktive (differente) Elektrode in einem Periode-Impuls-Betrieb. Dieser HF-Strom sorgt für die Erzeugung von Niedertemperaturplasma mit einer Glimmentladung unter Wasserdampf an der Spitze des distalen Endes der aktiven Elektrode. Der Wasserdampf entsteht dabei aus der Wasserkomponente der leitfähigen Flüssigkeit, indem diese Flüssigkeit im distalen Teil der Arbeitselektrode durch den HF-Strom schnell erhitzt wird. Gleichzeitig mit der Erhitzung erfolgt eine Generierung einer vorgegebenen Menge von Wasserstoffperoxid im Kontaktbereich der leitfähigen Flüssigkeit und der Oberfläche des biologischen Gewebes im Behandlungsgebiet.

Dabei sorgt die Einrichtung für eine Kontrolle und eine automatische Parametersteuerung der Elektroimpulsbehandlung und der bakteriziden Behandlung gemäß dem Ausgangszustand des Entzündungsherds, dem Charakter und der Art des biologischen Gewebes und sowie gemäß der eingestellten Eintauchtiefe der aktiven Elektrode in das biologische Gewebe. Dabei werden die optimalen Verläufe (Modi) für die Elektroimpulseinwirkung und die bakterizide Einwirkung sichergestellt.

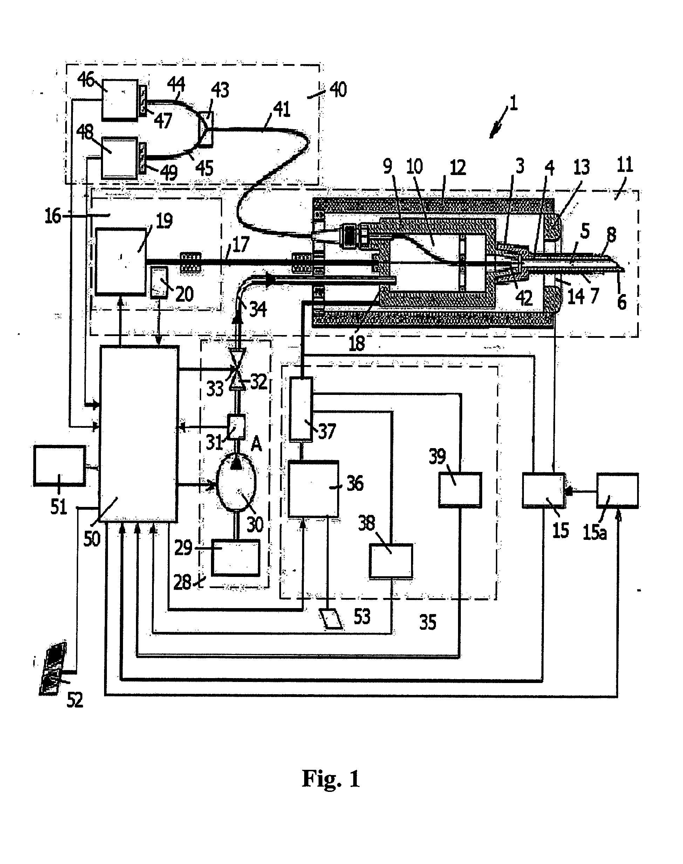

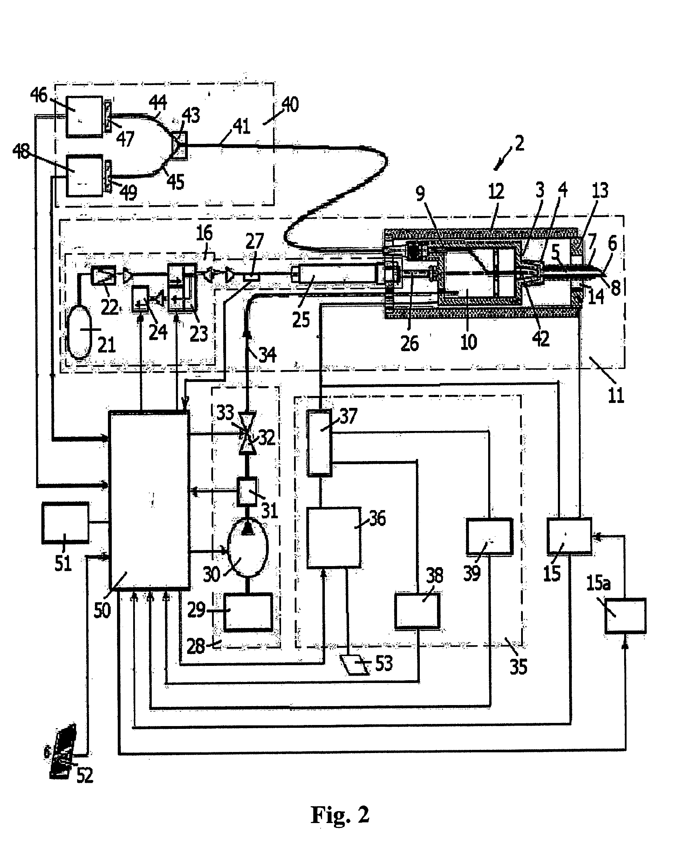

Die elektrochirurgische Einrichtung zur invasiven Elektrostimulation bei der Behandlung von Entzündungen gemäß der Erfindung ist nachfolgend in zwei Ausgestaltungen gemäß Fig. 1 und Fig. 2 dargestellt.

Dabei weisen die Einrichtung 1 (Fig. 1) und die Einrichtung 2 (Fig. 2) gemäß der Erfindung eine aktive (differente) Elektrode 3 mit einem distalen Ende 4 auf. Die aktive Elektrode 3 ist abnehmbar und nadelförmig ausgebildet und hat einen Innenkanal 5 und eine Spitze 6 sowie einen Isoliermantel 7 auf der Außenoberfläche der Nadel über die Länge ihres distalen Endes 4 bis auf das offene Ende 8 des distalen Endes 4 mit der Spitze 6. Die aktive Elektrode 3 hat auch ein proximales Ende 9 mit einem Behälter 10 zur Aufnahme der leitfähigen Flüssigkeit A. Dabei ist die aktive (differente) Elektrode 3 für den Einsatz im Applikator 11 angepasst.

Der Applikator 11, wie in Fig. 1, 2 abgebildet, hat ein Gehäuse 12 aus einem Nichtleiterwerkstoff zum Einbau der aktiven Elektrode 3 mit austauschbarer Nadel des distalen Endes 4 der Elektrode 3. Das Gehäuse 12 ist mit einem leitfähigen Kontaktflansch 13 versehen, um den elektrischen Kontakt des Applikators 11 mit dem Körper des Patienten sicherzustellen. Der Kontaktflansch 13 hat ein Mittenloch 14, um darin das distale Ende 4 der aktiven Elektrode 3 locker zu platzieren, wenn die aktive Elektrode 3 aus dem Gehäuse 12 über das Loch 14 des Kontaktflansches 13 in die Arbeitsstellung um ihre Eintauchtiefe in das biologische Gewebe hinausfährt. Die Einrichtung enthält einen Berührungssensor 15, welcher den Kontakt zwischen der Spitze 6 der genannten aktiven Elektrode 3 mit der Körperoberfläche des Patienten signalisiert. Der Berührungssensor 15 ist von einer AC-Stromversorgung 15a gespeist und sorgt für die Registrierung des Stroms, welcher zwischen dem Kontaktflansch 13 und der Spitze 6 der aktiven Elektrode 3 bei ihrer gegenseitigen Anordnung auf der Oberfläche des Körpers des Patienten fließt. Der Kontaktflansch 13 kann z. B. in Form einer Buchse, wie in Fig. 1 dargestellt, oder in Form einer Platte, wie in Fig. 2 dargestellt, ausgebildet sein. Dabei ist der Applikator 11 mit einem Antriebssystem 16 der aktiven Elektrode 3 und einem Weggeber der Elektrode 3 zur Signalisierung ihrer Verschiebung entlang des Gehäuses 12 des Applikators 11 versehen.

Das Antriebssystem 16 gemäß der Erfindung muss die Verschiebbarkeit der aktiven Elektrode 3 um eine Strecke erlauben, welche ihr Ausziehen aus dem Applikator 11 um die vorgegebene Eintauchtiefe des distalen Endes 4 der Elektrode 3 in das biologische Gewebe sicherstellt. Dieses Antriebssystem 16 kann z. B. mit einem mechanischen Antrieb, wie in Fig. 1 dargestellt, oder mit einem Druckluftantrieb, wie in Fig. 2 dargestellt, ausgerüstet sein.

Das in Fig. 1 dargestellte Antriebssystem 16 weist eine Zugstange 17 und einen Weggeber 20 auf. Die Zugstange 17 ist mit der Stirnseite 18 des proximalen Endes 9 der aktiven Elektrode 3 und mit einem Linear-Schrittmotor 19 verbunden. Der Weggeber 20 der aktiven Elektrode 3 ist auf der Zugstange 17 angeordnet und dient zur Angabe der Verschiebungsstrecke der aktiven Elektrode 3 von der Ausgangsstellung zum Zeitpunkt, wenn die Spitze 6 die Körperoberfläche des Patienten berührt, bis die maximale vorgegebene Eintauchtiefe der Spitze 6 in das biologische Gewebe erreicht ist.

Das in Fig. 2 dargestellte Antriebssystem 16 enthält einen Druckluftspeicher 21, ein Getriebe 22, einen elektrisch gesteuerten Verteiler 23 mit einem steuerbaren Ventil 24 und einen Druckluftzylinder 25 mit einem Kolben, der mittels einer Stange 26 mit der Stirnseite 19 des proximalen Endes 9 der aktiven Elektrode 3 gekoppelt ist, und einen auf der Stange 26 montierten Weggeber 27 der aktiven Elektrode 3. Der Weggeber 27 signalisiert die Verschiebung der aktiven Elektrode 3 aus ihrer Ausgangsstellung zum Zeitpunkt der Berührung zwischen der Spitze 6 der aktiven Elektrode 3 und der Körperoberfläche des Patienten um eine Strecke, bis die maximale vorgegebene Eintauchtiefe der Spitze 6 in das biologische Gewebe erreicht ist.

Die Einrichtungen 1 und 2 (Fig. 1, 2) enthalten ein System 28 für die Zuführung einer leitfähigen Flüssigkeit A gemäß der Erfindung. Dieses System 28 sorgt für die Zuführung einer vorgegebenen Menge der leitfähigen Flüssigkeit A innerhalb der Einführungsdauer des distalen Teils 4 der aktiven Elektrode 3 in das biologische Gewebe. Die Zuführung der vorgegebenen Menge der leitfähigen Flüssigkeit A erfolgt über einen Behälter 10 des proximalen Endes 9 der Elektrode 3 und den Innenkanal 5 der Nadel des distalen Endes 4 der aktiven Elektrode 3 mit einer vorgegebenen Zuführungsgeschwindigkeit. Diese Geschwindigkeit ist erforderlich, um rings um den distalen Teil 4 der aktiven Elektrode 3 bei ihrem Eintauchen ins biologische Gewebe einen Pufferbereich zu bilden. Dieser Pufferbereich enthält die leitfähige Flüssigkeit A in einer vorgegebenen Menge, welche den Kontakt zwischen der aktiven Elektrode 3 und dem biologischen Gewebe verhindert. Der Pufferbereich 54 hat eine vorgegebene Größe entsprechend dem Umfang des im biologischen Gewebe um die Tiefe des Behandlungsgebiets eingetauchten distalen Teils 4 der Elektrode 3.

Dabei kann das Zuführungssystem 28 der leitfähigen Flüssigkeit A einen Behälter 29 zur Aufnahme der leitfähigen Flüssigkeit A unter Druck, eine Pumpe 30, einen Druckgeber 31, ein elektrisch gesteuertes Ventil 32 mit einem Durchflussmesser 33 und eine Leitung 34 für die Zuführung der leitfähigen Flüssigkeit A in den Behälter 10 der aktiven Elektrode 3 enthalten. Dabei kann die Leitung 34 in Form eines flexiblen Kapillarrohrs ausgebildet sein. Ein Ende des Kapillarrohrs ist im Behälter 10 des proximalen Endes 9 der Elektrode 3 befestigt.

Die Einrichtungen 1 und 2 (Fig. 1, 2) enthalten ein Hochfrequenz- Stromversorgungssystem 35 gemäß der Erfindung. Es stellt eine HF-Stromversorgung des distalen Endes 4 der aktiven Elektrode 3 in einem Periode-Impuls-Betrieb sicher. Die dabei anstehende Spannung ist für die Generierung eines Niedertemperaturplasmas mit einer Glimmentladung am Ende der Spitze 6 des distalen Endes 4 der aktiven Elektrode 3 im Pufferbereich 54 für die Erwärmung der leitfähigen Flüssigkeit A, für die Dampfbildung und ihre Ionisation ausreichend. Dabei entsteht im Pufferbereich 54 eine solche Menge an Wasserstoffperoxid, welche für eine Elektrostimulation und eine bakterizide Behandlung des biologischen Gewebes im Behandlungsgebiet während der Herstellung des stabilen Plasmabetriebs und der nachfolgenden Verschiebung der Nadel des distalen Endes 4 der Elektrode 3 entlang des Behandlungsgebiets beim Herausziehen der Nadel aus dem Eintauchbereich ausreichend ist. Die Verschiebungsstrecke ist dabei kürzer als die vorgegebene Eintauchtiefe.

Wie in den Fig. 1 und 2 dargestellt ist, kann das Hochfrequenz- Stromversorgungssystem 35 gemäß der Erfindung Folgendes aufweisen:

- einen Hochfrequenz-Impulsgenerator 36,
- einen Stromgeber 37 und
- einen Integrator 38 zur Berechnung der an die Spitze 6 der Elektrode 3 angelegten Elektrizitätsmenge,
- einen Stromoberwellenanalysator 39, welcher das Vorhandensein der dritten Oberwelle und der Fundamentalschwingung im generierten elektrischen Signal überprüft. Die genannten Oberwellen kennzeichnen den Plasma-Betrieb des Generators 36,
- einen Spektralanalysator 40 für die Plasma-Strahlung. Der Spektralanalysator 40 überwacht die Plasmatemperatur sowie den Plasmabetrieb je nach Vorhandensein und Intensität der Emissionslinien der angeregten Natrium- oder Wasserstoffatome im Spektrum,

- einen Lichtleiter 41, wobei ein Ende 42 des Lichtleiters 41 im Innenkanal 5 der Nadel der aktiven Elektrode 3 angeordnet ist.

Dabei kann als Hochfrequenz-Impulsgenerator 36 ein HF-Generator eingesetzt sein, welcher eine elektrische Stromerzeugung mit einer Hauptträgerfrequenz im Frequenzbereich von 0,10 MHz bis 20,00 MHz und mit der Möglichkeit einer Modulationsparameterregelung sicherstellt, und zwar:

- mit einem Impulspausenverhältnis im Bereich von 1 bis 10,
- mit einer Impulsfolgefrequenz im Bereich von 1 kHz bis 20 kHz und
- mit einer Spannungsimpulshöhe bei fehlender Belastung im Bereich von 100 V bis 400 V.

Der Spektralanalysator 40 für die Plasma-Strahlung (Fig. 1, 2) gemäß der Erfindung kann Folgendes aufweisen:

- einen V-förmigen Verzweiger 43, der optisch mit dem genannten Lichtleiter 41 verbunden ist. Der Verzweiger 43 sorgt für die Aufteilung der Lichtemission des Plasmas in zwei optische Kanäle 44 und 45,
- einen lichtempfindlichen Sensor 46, der mit einem Lichtfilter 47 zur Aussonderung der Na- Linie von 337,1 nm oder der Wasserstoffatomlinie $H_\beta$ von 486,1 nm versehen ist, und
- einen lichtempfindlichen Sensor 48, der mit einem Lichtfilter 49 zur Aussonderung der Na-Linie von 588,9 nm oder der Wasserstofflinie $H_\alpha$ von 656,3 nm versehen ist.

Dabei enthalten die Einrichtungen 1 und 2 eine Steuerungseinheit 50 mit einem Steuerschalter, der elektrisch mit folgenden Komponenten verbunden ist:

- einem Berührungssensor 15 des Applikators 11 (Fig. 1, 2),
- einem Weggeber 20 (Fig. 1) oder einem Weggeber 27 (Fig. 2) des Antriebssystems 16,
- einem Druckgeber 31, einem elektrisch gesteuerten Ventil 32 und einer Pumpe 30 des Systems 28 für die Zuführung der leitfähigen Flüssigkeit A (Fig. 1, 2),
- einem HF-Impulsgenerator 36, einem Stromgeber 37 mit einem Integrator 38, einem Stromoberwellenanalysator 39, einem Spektralanalysator 40 für Plasma-Strahlung des Hochfrequenz-Stromversorgungssystems 35 (Fig. 1, 2).

Dabei ist die Steuerungseinheit 50 gemäß der Erfindung an eine Stromversorgung 51 angeschlossen und enthält wenigstens:

- einen Mikrorechner, der ebenfalls mit der steuerbaren Sammeleinrichtung, einer Einrichtung zum Empfang und Senden der elektrischen Signale von und zu den genannten Sensoren, Gebern und Systemen der Einrichtung (in der Zeichnung nicht abgebildet), verbunden ist.
- eine Fußtaste 52 zur Fernein- und -ausschaltung der Einrichtung.

Die Steuerungseinheit 50 gemäß der Erfindung stellt wenigstens Folgendes sicher:

- die Berechnung der Betriebsparameter der genannten Systeme der Einrichtung. Diese Betriebsparameter sind optimale Kenndaten für die Elektroimpulsbehandlung und die bakterizide Behandlung des biologischen Gewebes je nach den durch den Chirurgen gewählten Kriterien. Diese Kriterien schließen die Art und den Charakter des biologischen Gewebes im Behandlungsgebiet, den Zustand des Entzündungsherds, die Elektrodengröße, die Eintauchtiefe der aktiven Elektrode 3 in das biologische Gewebe und die Tiefe des Behandlungsbereichs des biologischen Gewebes ein,
- die Erstellung und Online-Aktualisierung (Aufrechterhaltung des aktiven Zustands) der Datenbanken der Parameter der genannten Systeme der Einrichtung, welche für die Erzeugung von Steuerbefehlen erforderlich sind, wobei diese Befehle das Ein- und/oder Ausschalten bzw. die Aktualisierung der Parameter oder die Unterbrechung bzw. Sperrung von einzelnen Operationen oder die Not-Abschaltung der Einrichtung verursachen,
- das Weiterleiten der Steuerbefehle an die genannten Sensoren, Geber und Systeme der Einrichtung,
- die Überwachung der Einhaltung der genannten Parameter aufgrund der Auswertung der elektrischen Signale von den genannten Systemen der Einrichtung,
- die digitale Verarbeitung der empfangenen elektrischen Signale und
- die Visualisierung der Betriebsparameter der Systeme der Einrichtung anhand graphischer und/oder Textmittel und die Anzeige des Einrichtungsbetriebs einschließlich der Anzeige der Alarm-Zustände im Betrieb der Einrichtung und solcher Situationen, die gesundheitsschädlich oder gefährlich für die Patienten sind.

Dabei ist die automatische Steuerungseinheit 50 gemäß der Erfindung für die Erstellung von wenigstens folgenden Datenbanken angepasst:

- die HF- Impulsspannung, welche die Funktion des Stromgebungssystems 35 im Plasma-Erzeugungsbetrieb je nach Einflussparametern sicherstellt,
- die Plasmatemperatur je nach dem Verhältnis der Strahlenintensitäten der Na- und Wasserstofflinien beim Übergang auf höhere Energieebenen,
- den Gesamtwert der durch das Plasma durchlaufenden Ladung,
- die Elektrodenverfahrgeschwindigkeit je nach der Größe des durch das Plasma fließenden Stroms,
- die erforderlichen Maße des Pufferbereichs je nach dem Durchmesser der aktiven Elektrode 3 und der Tiefe des Behandlungsgebiets,
- die Menge der leitfähigen Flüssigkeit A, welche für die Bildung des Pufferbereichs je nach den Abmessungen des Pufferbereichs und dem Durchmesser der Nadel der aktiven Elektrode 3 erforderlich ist;
- die Zuführungsgeschwindigkeit der leitfähigen Flüssigkeit A entsprechend der Menge der leitfähigen Flüssigkeit A im Pufferbereich und der Verschiebungsgeschwindigkeit der Elektrode 3.

Dabei ist der Mikrorechner der Steuerungseinheit 50 gemäß der Erfindung für die Ermittlung der aktuellen Geschwindigkeit $C_{equtv}$ der Erzeugung von Wasserstoffperoxid in der leitfähigen Flüssigkeit A im Pufferbereich je nach der durch Plasma durchgegangenen Elektrizitätsmenge q angepasst. Die Geschwindigkeit $C_{equtv}$ ist wie folgt zu berechnen:

$$C_{equtv} = F\left[1 - \exp\left(-\frac{G_0 kq}{V}\right)\right] / k$$

,

wobei

q für die Elektrizitätsmenge in Faraday,
$G_0$ für die Menge von Grammäquivalenten von Wasserstoffperoxid, welche ursprünglich pro Faraday der durchgelaufenen Elektrizität (Anfangsstromausbeute) gebildet werden,
V für die Menge der leitfähigen Flüssigkeit A im Pufferbereich,
k für die Geschwindigkeitszahl der Wasserstoffperoxid-Zersetzungsreaktion,
F für die Erfahrungszahl, welche von den Kennwerten der leitfähigen Flüssigkeit A abhängt,

stehen.

Die Einrichtungen 1 und 2 enthalten auch eine mit einem HF-Impulsgenerator 36 verbundene passive Elektrode 53. Die passive Elektrode 53 ist für die Anordnung am Körper des Patienten in einem Gebiet gemäß den ärztlichen Indikationen für den Patienten und so nahe wie möglich an das Behandlungsgebiet angepasst.

Dabei ist in der elektrochirurgischen Einrichtung gemäß der Erfindung als leitfähige Flüssigkeit A eine 0,9%ige physiologische Kochsalzlösung verwendet.

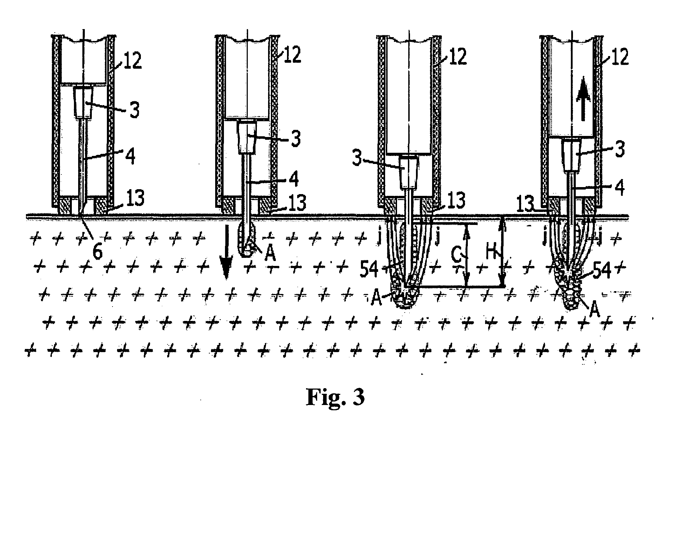

Der Ablauf der durchgeführten Elektrostimulation und der bakteriziden Behandlung ist schematisch in den Fig. 3 a, b, c, d abgebildet. Der Betrieb der Einrichtungen 1 und 2 erfolgt schrittweise. Dabei werden der Anfang und der Abschluss jedes Schritts vor der Umschaltung der Einrichtung zum nächsten Schritt überwacht.

Schritt 1. Vorbereitung der Einrichtungen zum Betrieb (Fig. 1, 2, 3a):

Beim Einschalten der Stromversorgung 51 wird der Mikrorechner der Steuerungseinheit 50 aktiviert.

Nach dem Befehl "Vorgabe der Kennwerte und Betriebsarten" stellt der Mikrorechner dem Chirurgen anhand der vorhandenen Software (Programme) automatisch die in der Datenbank des Mikrorechners gespeicherten Kriterien zur Verfügung. Diese Kriterien kennzeichnen die Besonderheiten der durchzuführenden Behandlung und umfassen die Unterschiede in der Art und dem Charakter des biologischen Gewebes im Behandlungsgebiet, die Übereinstimmung des Zustands des zu behandelnden Entzündungsherds mit den Kriterien aus der Datenbank, die Größen des austauschbaren distalen Teils 4 der aktiven Elektrode 3, die Eintauchtiefe der aktiven Elektrode 3 in das biologische Gewebe und die Tiefe des Behandlungsbereichs des biologischen Gewebes. Der Chirurg kann dabei solche Kriterien auswählen, die bei der vorstehenden Prozedur der Elektrostimulation zu benutzen sind.

Der Mikrorechner berechnet oder wählt aus der Datenbank solche Betriebsparameter der Systeme der Einrichtung aus, welche für die Elektroimpulsbehandlung und die bakterizide Behandlung des biologischen Gewebes je nach den durch den Chirurgen gewählten Kriterien optimal geeignet sind.

Der Mikrorechner erstellt die Gesamtheit der Betriebsparameter der Einrichtung, die für die Arbeit der Einrichtung im Optimalbetrieb bei den durch den Chirurgen eingestellten Kriterien erforderlich ist. Der Mikrorechner sorgt auch für die Generierung von solchen Steuerbefehlen, die an die gesteuerte Sammeleinrichtung gesendet werden und das Aktivieren/Deaktivieren oder die Nachbesserung der Ausgangkennwerte verschiedener Systeme der Einrichtung verursachen, oder zum Abbrechen oder Sperren von einzelnen Vorgängen/Operationen oder zur Not-Ausschaltung der Einrichtung führen.

Die steuerbare Sammeleinrichtung sendet Steuerbefehle für die Aktivierung von Systemen der Einrichtung und der oben genannten mit der Steuerungseinheit 50 verbundenen Sensoren und Gebern einschließlich der Fußtaste 52 zur Fernsteuerung, wobei der Ausgangszustand der genannten Sensoren, Geber und Systeme vor dem Betriebsanfang der Einrichtung im Elektrostimulation-Modus festgehalten ist.

Die Einrichtung ist nun bereit, in den Betrieb nach Schritt 2 zu wechseln.

Die Beendigung von Schritt 1 wird zum Beispiel durch die Darstellung der Gesamtheit der eingestellten Kennwerte auf dem Bildschirm und die Bereitschaftsanzeige jedes der Systeme der Einrichtung für die Arbeit im genannten vorgegebenen Modus signalisiert.

Schritt 2. Einführung der aktiven Elektrode 3 (Fig. 1, 2, 3b, c):

Der Applikator 11 der Einrichtung 1, 2 sowie der Kontaktflansch 13 sind an der Hautoberfläche des Patienten im Behandlungsgebiet in der Nähe des Entzündungsherds, wie in Fig. 3 dargestellt ist, gemäß den ärztlichen Indikationen angeordnet. Der Abstand zur vermutlichen Außengrenze des Entzündungsherds (oder zum Entzündungsherd) beträgt 5 - 10 mm.

Nach dem Befehl von der Steuerungseinheit 50 erzeugt der Generator 15a ein schwaches, max. 10 mA starkes, elektrisches Signal mit einer Frequenz im Bereich von 0,5 - 50,0 kHz. Dieses Signal wird an die Nadel der aktiven Elektrode 3 während des gesamten Betriebs der Einrichtung gegeben. Der Berührungssensor 15 ist aktiviert.

Die Funktionsweise der Einrichtungen 1 und 2 beim Schritt 2 ist weiter anhand der Funktionsbeispiele der Einrichtung 1 mit einem mechanischen Elektrodenantriebssystem (Beispiel 1 von Schritt 2) und der Einrichtung 2 mit dem pneumatischen E-lektrodenantriebssystem 3 (Beispiel 2 von Schritt 2) beschrieben.

Beispiel 1

Nach dem Befehl der Steuerungseinheit 50 «Einführung der Nadel» schaltet sich das Antriebssystem 16 ein. Dabei läuft der Linear-Schrittmotor 19 an. Die Zugstange 17 wird aus der End-Grundstellung (Fig. 1, links) verschoben und stößt dabei gegen die Stirnseite 18 des proximalen Endes 9 der Elektrode 3 nach rechts. Damit ist die Verschiebung der Elektrode 3 entlang des Gehäuses 12 des Applikators 11 sichergestellt, so dass das distale Ende 4 in Richtung des Kontaktflansches 13 befördert ist.

Beispiel 2

Nach dem Befehl der Steuerungseinheit 50 «Einführung der Nadel» wird das Getriebe 22 eingeschaltet. Der elektrisch gesteuerte Verteiler 23 mit einem steuerbaren Ventil 24 ist geöffnet. Die Luft kommt aus dem pneumatischen Speicher 21 in den Druckluftzylinder 24 mit dem Kolben und der Stange 26 und beaufschlagt somit den Kolben mit Druck. Die Stange 26 wird dadurch in Bewegung versetzt und betätigt die damit verbundene Stirnseite 18 des proximalen Endes 9 der aktiven Elektrode 3. Dadurch wird die Verschiebung der Elektrode 3 entlang des Gehäuses 12 des Applikators 11 sichergestellt, so dass das distale Ende 4 in Richtung des Kontaktflansches 13 befördert ist.

Danach hält der vorher aktivierte Berührungssensor 15 in den dargestellten Ausgestaltungen der Einrichtungen 1 und 2 den Zeitpunkt fest, bei dem der elektrische Strom zwischen dem Kontaktflansch 13 und der Spitze 6 der Nadel des distalen Endes 4 der Elektrode 3 über der Hautoberfläche entsteht. Sobald die Spitze 6 der Elektrode 3 das biologische Gewebe berührt, wird der Stromkreis geschlossen, und der Berührungssensor 15 notiert den vorliegenden Kontakt.

Nach dem Eingang des Signals vom Berührungssensor 15 gibt die Steuerungseinheit 50 über den Steuerschalter gleichzeitig das Ein-Kommando für das System 28 für die Zuführung der leitfähigen Flüssigkeit A ab, um in den Betrieb «Herstellung von Pufferbereich» zu wechseln. Gleichzeitig wird ein Kommando zur Aktivierung des Weggebers 20 (Fig. 1) (oder des Weggebers 27 (Fig. 2)) des Antriebssystems 16 abgegeben, wobei die Null-Stellung des distalen Endes 4 der Elektrode 3 bis zu ihrem Eintauchen in das biologische Gewebe festzuhalten ist. Gleichzeitig wird auch ein Befehl zum Ausfahren des distalen Endes 4 nach außen an den Kontaktflansch 13 des Applikators 11 gegeben.

Nach dem Empfang des Ein-Kommandos für das System 28 für die Zuführung der leitfähigen Flüssigkeit A und die Arbeit im Betrieb «Herstellung von Pufferbereich» in Übereinstimmung mit den durch die Steuerungseinheit 50 vorgegebenen Kennwerten läuft die Pumpe 30 an. Sie fördert die Flüssigkeit A aus dem Behälter 29 in die Leitung 34. Das elektrisch gesteuerte Ventil 32 mit dem Durchflussmesser 33 stellt die Beförderung der vorgegebenen Menge der leitfähigen Flüssigkeit A mit vorgegebener Geschwindigkeit in die Leitung 34, in den Behälter 10 des proximalen Endes 9 der Elektrode 3 und über den Innenkanal 5 der Nadel der Elektrode 3 in den Behandlungsbereich hin sicher. Dies erfolgt je nach Vorschub der Nadel der Elektrode 3 um die vorgegebene Eintauchtiefe, welche durch den Weggeber 20 (oder den Weggeber 27) überwacht wird. Dabei überwacht der Druckgeber 31 den vorgegebenen Druck. Aufgrund der Daten vom Weggeber 20 der Einrichtung 1 (oder vom Weggeber 27 der Einrichtung 2) sowie der Daten über die Zuführungsgeschwindigkeit der leitfähigen Flüssigkeit A berechnet die Steuerungseinheit 50 die Ist-Menge der geförderten leitfähigen Flüssigkeit A und ermittelt in der Echt-Zeit den Umfang (die Größe) des je nach Vorschub der Nadel der Elektrode 3 erzeugten Pufferbereichs 54 (Fig. 3b, c). Gleichzeitig wird überprüft, ob diese Größe den eingestellten Kennwerten des Pufferbereichs 54 entspricht.

Nach der Beendigung von Schritt 2 sind der Behälter 10 des proximalen Endes 9 der Elektrode 3 und der Innenkanal 5 der Nadel des distalen Endes 4 der Elektrode 3 mit leitfähiger Flüssigkeit A gefüllt. Die Spitze 6 der Nadel des distalen Endes 4 der Elektrode 3 befindet sich in ihrer max. Eintauchtiefe. Um das distale Ende 4 der im biologischen Gewebe eingetauchten Elektrode 3 herum ist der Pufferbereich 54 mit der vorgegebenen Größe ausgebildet. Das Antriebssystem 16 hat den Status «Verschiebung der Elektrode ist abgeschlossen». Der Weggeber 20 (oder der Weggeber 27) ist auf Null gesetzt. Das System 28 für die Zuführung der leitfähigen Flüssigkeit A ist ausgeschaltet. Der Berührungssensor 15 ist aktiviert.

Schritt 3. Betriebsart für die Bildung der stabilen Plasmaentladung (Fig. 1, 2, 3c):

Der Empfang des Signals vom rückgesetzten Weggeber 20 (oder vom rückgesetzten Weggeber 27), setzt voraus, dass das System 28 für die Zuführung der leitfähigen Flüssigkeit A ausgeschaltet ist und dass die Daten über die Größe des erzeugten Pufferbereichs 54 vorliegen. Dann gibt die Steuerungseinheit 50 einen Befehl ab, um den HF-Generator 36 einzuschalten und den Stromgeber 37, den Integrator 38, den Stromoberwellenanalysator 39 und den Spektralanalysator 40 für die Plasma-Strahlung zu aktivieren.

Der Generator 36 legt HF-Strom an das distale Ende 4 der Elektrode 3 an, welche mit leitfähiger Flüssigkeit A gefüllt und in das biologische Gewebe eingetaucht und von dem Pufferbereich 54 umgeben ist.

Der Generator 36 erzeugt den Strom in einem Periode-Impuls-Betrieb. Seine Parameter sind durch die Steuerungseinheit 50 im Schritt 1 als optimal geeignet festgelegt .Dies erfolgt also unter Berücksichtigung der Bedingungen, welche für die Generierung des Niedertemperaturplasmas mit der Glimmentladung am Ende der Spitze 6 des distalen Endes 4 der aktiven Elektrode 3 innerhalb des Pufferbereichs 54 erforderlich sind, um eine solche Menge an Wasserstoffperoxid zu produzieren, die für die bakterizide Behandlung des biologischen Gewebes im Einwirkungsbereich ausreichend ist. Gemäß der Erfindung erfolgt die Stromerzeugung mit einer Hauptträgerfrequenz, die im Frequenzbereich von 0,10 MHz bis 20,00 MHz und mit der Möglichkeit der Modulationsparameterregelung gewählt ist, die

-    mit einem Impulspausenverhältnis im Bereich von 1 bis 10,
-    mit einer Impulsfolgefrequenz im Bereich von 1 kHz bis 20 kHz und
-    mit einer Spannungsimpulshöhe bei fehlender Belastung im Bereich von 100 V bis 400 V wählbar ist.

Der Stromgeber 37 verfolgt dabei die aktuellen Stromstärken. Der Integrator 38 berechnet die Elektrizitätsmenge, welche durch die Spitze 6 der Elektrode 3 durchgelaufen ist. Der Stromoberwellenanalysator 39 erkennt das Vorhandensein der dritten Stromoberwelle und der Fundamentalschwingung im HF-Stromkreis, welche den Plasma-Betrieb der Einrichtung kennzeichnen. Das Endstück 42 des Lichtleiters 41 ist im Innenkanal 5 der Nadel der aktiven Elektrode 3 angeordnet. Die Plasmastrahlung kommt von der Spitze 6 der Nadel des distalen Endes 4 der Elektrode 3 über den Lichtleiter 41 und über den V-Verzweiger 43 in zwei optische Kanäle 44 und 45, d. h. in den lichtempfindlichen Sensor 46 und in den lichtempfindlichen Sensor 48. Der V-Verzweiger 43 teilt die Lichtemission des Plasmas auf. Der lichtempfindliche Sensor 46 ist mit einem Lichtfilter 47 zur Aussonderung der Na-Emissionslinie bei einer Wellenlänge von 337,1 nm oder der Wasserstoffatomlinie $H_\beta$ bei einer Wellenlänge von 486,1 nm versehen. Der lichtempfindliche Sensor 48 ist mit dem Lichtfilter 49 zur Aussonderung der Na-Emissionslinie bei einer Wellenlänge von 588,9 nm oder der Wasserstofflinie $H_\alpha$ bei einer Wellenlänge von 656,3 nm versehen. Anhand der registrierten Na- und Wasserstoffemissionslinien kann der Anfang des Plasmabildungs-vorgangs am Ende der Spitze 6 überwacht werden. Die Plasmatemperatur kann anhand der relativen Intensität der Emissionslinien der Na- oder Wasserstofflinien im Strahlungsspektrum des Plasmas überwacht werden.

Die Steuerungseinheit 50 berechnet die Geschwindigkeit der Erzeugung von Wasserstoffperoxid und korrigiert ggf. die Kennwerte des Periode-Impuls-Betriebs des Generators 36.

Nach dem Abschluss von Schritt 3 sind der Generator 36, der Stromgeber 37, der Integrator 38, der Oberwellenanalysator 39 und der Spektralanalysator 40 für Plasma-Strahlung eingeschaltet. Die Plasmaerzeugung verläuft stabil. Die Plasma-Parameter stimmen mit den Vorgaben überein. Die Plasmagastemperatur beträgt max. 80°C. Die Spitze 6 der Nadel der Elektrode 3 mit angezündetem Plasma ist im biologischen Gewebe um die vorgegebene Eintauchtiefe H eingetaucht, wobei diese Tiefe die Tiefe des Behandlungsgebiets C überschreitet. Die Steuerungseinheit 50 zeigt den Betriebszustand «Plasma stabil» an.

Schritt 4. Betriebsart Elektroimpulsbehandlung und bakterizide Behandlung des biologischen Gewebes (Fig. 1, 2, 3d):

Nach dem Ein-Kommando der Steuerungseinheit 50 läuft das Antriebssystem 16 der Elektrode 3 an. Das distale Ende 4 der Elektrode 3 beginnt seine Bewegung zum Gehäuse 12 des Applikators 11. Gleichzeitig wird der elektrische HF-Strom an die Spitze 6 des distalen Endes 4 der aktiven Elektrode 3 in einem Periode-Impuls-Betrieb angelegt. Dieser Periode-Impuls-Betrieb wird im Rahmen von Schritt 3 festgelegt und bewerkstelligt die Erzeugung des Niedertemperaturplasmas mit der Glimmentladung an der Spitze 6 des distalen Endes 4 der aktiven Elektrode 3.

Beim Brennen der Plasma-Glimmentladung kommt die Zersetzung von Wassermolekülen zustande. Das ist durch die dissoziative Anhaftung während der Bildung des negativ geladenen Ions $H_2O^-$ möglich. Dabei sind die aktiven Hauptteilchen in der oberflächennahen Schicht der leitfähigen Flüssigkeit A das Hydroxonium-Molekül OH' und die Solvatisierenelektronen. Die nachfolgende Dimerisation der OH'-Moleküle führt zur Bildung von Wasserstoffperoxid $H_2O_2$. Dies wirkt als stabiles Haupt-Oxydans, welches in Wasserlösungen der Elektrolyte unter Einwirkung von Plasma der elektrischen Entladung erzeugt wird.

Somit führt die Einwirkung der Plasma-Glimmentladung auf die Wasserlösungen des Elektrolyts zur Anreicherung der aktiven oberflächennahen Zone vom Elektrolyt mit starken Oxydans-OH'- und Wasserstoffperoxid-Molekülen. Dabei ist die Zersetzung von Wasserstoffperoxid in den biologischen Geweben von der Einwirkung auf das biologische Gewebe her ausschlaggebend.

Der mit der Einwirkung (Oxidation) der freien Atome, Radikale und Moleküle von Wasserstoffperoxid auf das Protein-Gefüge des biologischen Gewebes zusammenhängende Chemismus führt auch zu ihrer Destruktion. Dabei verursachen solche Vorgänge keine Destruktion von tief liegenden Schichten des biologischen Gewebes, sondern betreffen nur die oberflächennahe Schicht.

Die Störung der Molekularstruktur der oberflächennahen Schicht des biologischen Gewebes ruft die Aktivierung des Immunsystems des Körpers hervor. Das führt zum wirksamen Kupieren des Entzündungsherds und seiner Ausheilung.

Beim Schritt 4 wird das biologische Gewebe der beschriebenen plasmachemischen und bakteriziden Behandlung beim Herausziehen der Nadel aus dem Eintauchbereich H innerhalb der Bewegungsdauer der Nadel des distalen Endes 4 der Elektrode 3 entlang des Behandlungsgebiets C ausgesetzt. Das Behandlungsgebiet C weist eine vorgegebene Länge auf, die kleiner als die vorgegebene Eintauchtiefe ist. Dabei wird die Erzeugung der vorgegebenen Menge von Wasserstoffperoxid, wie oben beschrieben, überwacht. Dafür werden die aktuellen Geschwindigkeiten der Wasserstoffperoxiderzeugung und seine Mengen, die Stromintensität, die Kenndaten des Plasmabetriebs und die Bewegungsgeschwindigkeit der Nadel am distalen Ende 4 der Elektrode 3 entlang des Behandlungsgebiets berechnet. Damit ist die Kontaktdauer zwischen dem Plasma und der leitfähigen Flüssigkeit A im Pufferbereich 54 sowie die Kontaktdauer zwischen dem Wasserstoffperoxid und der Oberfläche des biologischen Gewebes im Behandlungsgebiet C sichergestellt, welche für eine wirksame Bearbeitung notwendig sind.

Die Mengenkontrolle des erzeugten Wasserstoffperoxids wird durchgeführt. Dafür ist die Menge der durchgelaufenen Elektrizität anhand der Integration der Stromkonstante des durch die Elektrode 3 durchgegangenen Stroms zu messen. Der Stromwert ist durch den Spannungsabfall am Stromgeber 37 (niederohmiger Widerstand) bestimmt.

Das biologische Gewebe muss die notwendige Menge an Wasserstoffperoxid speichern. Deswegen wird die Geschwindigkeit beim Herausziehen der Elektrode 3 aus dem Biogewebe durch die Kinetik der Wasserstoffperoxid-($H_2O_2$)-Erzeugung in der physiologischen Lösung unter der Entladungswirkung bestimmt und mit Hilfe eines Mikrorechners und eines Weggebers gesteuert.

Gemäß der Erfindung erfolgt das Plasma-Brennen am distalen Ende der Elektrode 3, wenn die Elektrode 3 herausgezogen wird, und hört auf, wenn die Spitze 6 sich am Ende des vorgegebenen Behandlungsgebiets C befindet, und zwar beim Austreten der Elektrodenspitze 6 an die Außenoberfläche des Körpers und vor der Entnahme der Spitze 6.

Der elektrische Kreis der Elektroimpuls-Plasma-Einwirkung durch die aktive Elektrode 3 wird an der passiven Elektrode 53 geschlossen.

Nach der Entnahme der Elektrode 3 und nach dem Signal vom Berührungssensor 15 über den Ausfall des Stroms wird der Integrator 38 vor der nächsten Einführung einer nadelförmigen Elektrode 3 in das biologische Gewebe zwangsläufig zurückgesetzt.

Die durchgeführten Versuche haben gezeigt, dass bei der Auswahl folgender Kriterien für die Ausführung der elektrostimulierenden und bakteriziden Behandlung des Pyodermie-Herds im Lendeabschnitt gewählt sind:

- Der Charakter (Art) des biologischen Gewebes um den Entzündungsherd herum ist: subkutaner Zellstoff.
- Zustand des Entzündungsherds: Es ist kein Eiterherd vorhanden.
- Die vorgegebene Eintauchtiefe der aktiven Elektrode 3 in das biologische Gewebe ist 5 mm.
- Die vorgegebene Tiefe des Behandlungsgebiets ist 3,5 mm.
- Der Durchmesser der Elektrodenadel ist 3 - 0,3 mm.

Die folgenden Betriebsdaten für den Generator 36 sind wie folgt festgelegt:

- die Betriebsfrequenz des Generators 2,64 MHz,
- die Impulsspannung 250 V,
- das Impulsverhältnis 7,
- die Impulsfolgefrequenz 5 kHz und
- die Menge des erzeugten Wasserstoffperoxids $5.10^{-6}$ Mol.

Dabei betrug die Eintauchgeschwindigkeit der Nadelspitze 6 des distalen Teils 4 der Elektrode 3 in das biologische Gewebe 0,5 cm/Sek. und die Auszugsgeschwindigkeit der Nadelspitze 6 des distalen Teils 4 der Elektrode 3 aus dem biologischen Gewebe betrug 0,1 cm/Sek. Die Behandlung wurde unmittelbar im Entzündungsherd vorgenommen. Dabei dauerte die Elektroimpulsbearbeitung insgesamt max. 2 Minuten (4 Applikationen).

Der Effekt - das Verschwinden von sichtbaren oder für den Patienten wahrnehmbaren Entzündungszeichen - wurde im Laufe von 7 Tagen erreicht.

Somit stellt die elektrochirurgische Einrichtung zur Behandlung von Entzündungen mittels invasiver Elektrostimulation gemäß der Erfindung die Bearbeitung des biologischen Gewebes im Entzündungsgebiet mittels des Hochfrequenzstroms im halbautomatischen Betrieb mit hohem Wirkungsgrad sicher. Dabei sorgt die Einrichtung auch für die Überwachung und die automatische Steuerung der Parameter der Elektroimpulsbehandlung und der bakteriziden Behandlung gemäß dem Ausgangszustand des Entzündungsherds und dem Charakter des biologischen Gewebes.

Bei der Entwicklung der Erfindung wurden Kriterien für den Wechsel von der Wärmebeanspruchung des Biogewebes zur plasmachemischen Einwirkung festgelegt, die Methoden für ihre Optimierung und Aufrechterhaltung ausgearbeitet und die Schwellwertbedingungen für das Entstehen der Plasma-Glimmentladung bei einer Spannungserhöhung auf der aktiven Elektrode (Stromoberwellenspitze im Stromkreis der Elektrode) erkannt.

Einem Fachmann im Bereich der Medizintechnik ist klar, dass die Einrichtung gemäß der Erfindung verschiedene Verbesserungen und Weiterbildungen haben kann, die allerdings nicht über den Schutzumfang der vorliegenden Erfindung hinausgehen. So kann zum Beispiel der Aufbau der Systeme der Einrichtung zwecks ihrer Kompaktheit bzw. Miniaturisierung vervollkommnet werden, um die Benutzerfreundlichkeit zu erhöhen und die Durchführung von gleichzeitiger Behandlung mehrerer Stellen mit mehreren Elektroden mit einem mehrfachen Applikator sicherzustellen. Ein solcher Applikator enthält mehrere Elektroden, die im Applikator in einem bestimmten Abstand zueinander (im Raster) aufgebaut sind. Die Einrichtung kann auch eine Speicherung von statistischen Daten in Bezug auf den Anwendungsnutzen von unterschiedlichen Bearbeitungsmodi zur Behandlung von verschiedenen Entzündungen und die Erstellung von Datenbanken ermöglichen.

Die elektrochirurgische Einrichtung zur Behandlung von Entzündungen mittels invasiver Elektrostimulation kann zur Behandlung von verschiedenen Entzündungen mit der Bearbeitung des biologischen Gewebes sowohl im Bereich um den Entzündungsherd herum als auch unmittelbar im Entzündungsherd angewendet werden. Sie kann mit Hilfe von üblichen bekannten verfahrenstechnischen Verfahren und Ausrüstungen durchgeführt werden. Die Einrichtung stellt die Überwachung und die Regelung der Einflussparameter innerhalb der für den Organismus optimalen Grenzwerte in Übereinstimmung mit ärztlichen Indikationen sicher. Dabei ist ein solcher Faktor wie ein subjektives Urteil über die Fachfertigkeiten und -kenntnisse des Chirurgen ausgeschlossen.

**Patentansprüche**

1. Elektrochirurgische Einrichtung zur Behandlung von Entzündungen mittels invasiver Elektrostimulation, mit einer aktiven (differenten) Elektrode (3) und einer passiven Elektrode (52), wobei die aktive Elektrode (3) in einem Applikator (11) eingebettet ist, und einen Innenkanal (5) für die Zuführung einer leitfähigen Flüssigkeit (A) in das Behandlungsgebiet (C) eines biologischen Gewebes sowie einen Isoliermantel (7) auf seiner Außenoberfläche über die Länge ihres distalen Teils (4) bis auf das offene Ende (8) des distalen Teils (4) sowie ein mit der aktiven Elektrode (3) verbundenes System zur HF-Stromversorgung (35) aufweist, wobei der HF-Strom die Erzeugung eines Niedertemperaturplasmas am Ende des distalen Teils (4) der aktiven Elektrode (3) in Anwesenheit der leitfähigen Flüssigkeit (A) sicherstellt, und wobei eine passive Elektrode (53) an den Körper des Patienten anlegbar ist,
**dadurch gekennzeichnet,**

**dass** die Einrichtung für die Durchführung von Elektrostimulation in der Nähe des Entzündungsherds mittels Destruktion der Molekularstruktur der oberflächennahen Schichten des biologischen Gewebes und gleichzeitiger bakterizider Behandlung des biologischen Gewebes beim Kontakt des biologischen Gewebes mit einem leitfähigen Pufferbereich (54) angepasst ist, wobei der Pufferbereich (54) das Plasma umringt und rings um das distale Ende (4) der aktiven Elektrode (3) ausgebildet ist,

**dass** die aktive Elektrode (3) dabei eine Spitze (6) aufweist und in das biologische Gewebe vorher eintauchbar ist, wobei die Eintauchtiefe die Tiefe des Behandlungsgebiets (C) mit der leitfähigen Flüssigkeit (A) überschreitet, dass die leitfähige Flüssigkeit (A) im Voraus und zwar beim Eintauchen des distalen Endes (4) dem Behandlungsgebiet (C) aus einem Behälter (10) zuführbar ist, welcher am proximalen Ende (9) der aktiven Elektrode (3) angeordnet ist,

**dass** die Einrichtung dabei die Durchführung der genannten Behandlung bei der Austauschung (Entnahme) der Elektrode (3) aus dem biologischen Gewebe sicherstellt und Folgendes aufweist:

- einen Applikator (11), der das Austauschen der aktiven Elektrode (3) nach draußen mit einer vorgegebenen Geschwindigkeit sicherstellt, wobei das Austauschen über die Stirnfläche des Applikators (11) in einem Abstand erfolgt, welcher seiner vorgegebenen Eintauchtiefe im biologischen Gewebe gleich ist, dass dabei der Berührungszeitpunkt zwischen der aktiven Elektrode (3) und der Körperoberfläche des Patienten erfasst ist und dieser Zeitpunkt als Anhaltspunkt für die Bestimmung der Eintauchtiefe dient, dass der Applikator (11) auch ein Austauschen (Entnahme) der Elektrode (3) aus dem biologischen Gewebe mit einer vorgegebenen Geschwindigkeit sicherstellt, wobei diese Geschwindigkeit die vorgegebene Behandlungsdauer des biologischen Gewebes innerhalb der Austauschstrecke des Elektrodeendstücks (4) und zwar entlang der Tiefe des Behandlungsgebiets (C) bis zur Grundstellung der Elektrode (3) sichert,

**dass** ein System (28) für die Zuführung einer leitfähigen Flüssigkeit (A)vorgesehen ist, das für die Zuführung einer vorgegebenen Menge der leitfähigen Flüssigkeit (A) über einen Behälter (29) und einen Innenkanal (5) der aktiven Elektrode (3) mit einer vorgegebenen Geschwindigkeit innerhalb der Einführungsdauer der aktiven Elektrode (3) in das biologische Gewebe sorgt,

**dass** die genannte vorgegebene Geschwindigkeit erforderlich ist, um rings um den distalen Teil (4) der aktiven Elektrode (3) bei ihrer Eintauchung in das biologische Gewebe einen Pufferbereich (54) zu erzeugen,

**dass** dabei der Pufferbereich (54) den unmittelbaren Kontakt zwischen dem Plasma und dem biologischen Gewebe verhindert und vorgegebene Maße in Übereinstimmung mit dem Umfang des distalen Endes (4) der Elektrode (3) aufweist, die in das biologische Gewebe in der Tiefe des Behandlungsgebiets (C) eingetaucht sind,

**dass** ein HF-Stromversorgungssystem (35) vorgesehen ist, welches eine HF-Stromversorgung der leitfähigen Flüssigkeit (A) innerhalb der Zeitspanne des Austauschens der Elektrode (3) aus dem Behandlungsgebiet (C) sicherstellt, dass das HF-Stromversorgungssystem (35) im Innenraum der aktiven Elektrode (3) eingerichtet ist,

**dass** die HF-Stromversorgung (35) in einem Periode-Impuls-Betrieb mit solchen Kennwerten erfolgt, welche die Erzeugung eines Niedertemperatur-Plasmas einer Glimmentladung innerhalb des Pufferbereichs (54) am Ende des unisolierten Distalteils der aktiven Elektrode (3) bewerkstelligt und die Produktion einer vorgegebenen Menge von Wasserstoffperoxid im Pufferbereich (54) sicherstellt, dass diese Menge von Wasserstoffperoxid für eine Stimulation und eine bakterizide Behandlung des biologischen Gewebes im Behandlungsgebiet (C) ausreicht,

**dass** eine automatische Steuerungseinheit (50) mit einem System zum Empfang und zur Übertragung von elektrischen Signalen von und zu den genannten Funktionssystemen der Einrichtung, einem Mikrorechner, einer Fußtaste (52) zur Fernein- und -ausschaltung der Einrichtung, einer Stromversorgung, einem Steuerschalter vorgesehen sind,

**dass** die automatische Steuerungseinheit (50) zum Ein- und Ausschalten der Funktionssysteme der Einrichtung sowie für eine Aktualisierung ihrer Betriebszustände sorgt und folgende Funktionen erfüllt:

- eine Auswahl der Betriebsparameter der Einrichtung je nach den durch einen Chirurgen gewählten Kriterien gemäß dem Charakter des biologischen Gewebes im Behandlungsgebiet (C), nach dem Zustand des Entzündungsherds, nach den Maßen des distalen Endes (4) der aktiven Elektrode (3), nach ihrer Eintauchtiefe im biologischen Gewebe und je nach der Tiefe des Behandlungsbereichs des biologischen Gewebes,

- eine Bildung und Online-Aktualisierung (Aufrechterhaltung des aktiven Zustands) der Datenbanken der Parameter der genannten Systeme der Einrichtung, welche für die Erzeugung von Steuerbefehlen erforderlich sind, wobei diese Befehle das Ein- und/oder Ausschalten bzw. die Aktualisierung der Parameter oder die Unterbrechung bzw. Sperrung von einzelnen Operationen oder eine Not-Abschaltung der Einrichtung verursachen,

- eine Weiterleitung der Steuerbefehle an die genannten Systeme der Einrichtung,

- eine Überwachung der Einhaltung der genannten Parameter aufgrund der Auswertung der elektrischen Signale von den genannten Systemen der Einrichtung,

- eine digitale Verarbeitung der empfangenen elektrischen Signale und

- eine Visualisierung der Betriebsparameter der Systeme der Einrichtung anhand graphischer und/oder Textmittel und eine Anzeige des Einrichtungsbetriebs.

2. Einrichtung nach Anspruch 1,
**dadurch gekennzeichnet,**
**dass** der Applikator (11) Folgendes aufweist:

- ein Gehäuse (12) aus einem Nichtleiterwerkstoff zur Aufnahme der aktiven Elektrode (3), wobei das Gehäuse (12) mit einem leitfähigen Kontaktflansch (13) für die Platzierung des Applikators (11) am Körper des Patienten versehen ist und ein Mittenloch (14) für eine lockere Aufnahme des distalen Teils (4) der aktiven Elektrode (3) beim Herausziehen der Elektrode (3) aus dem Gehäuse (12) des Applikators (11) in die Arbeitsstellung um die Eintauchtiefe der Elektrode (3) in das biologische Gewebe aufweist,
- einen Berührungssensor (15) zur Signalisierung der Berührung zwischen der Spitze (6) der genannten aktiven Elektrode (3) und der Körperoberfläche des Patienten, wobei dieser Berührungssensor (15) die Registrierung des Stroms sicherstellt, welcher zwischen dem Kontaktflansch (13) und der aktiven Elektrode (3) auftritt, wenn diese die Körperoberfläche des Patienten berühren, und
- ein Antriebssystem (16) der aktiven Elektrode (3) mit einem Weggeber (20) der aktiven Elektrode (3).

3. Einrichtung nach Anspruch 2,
**dadurch gekennzeichnet,**
**dass** das Antriebssystem (16) der aktiven Elektrode (3) eine Zugstange (17) und einen Weggeber (20) der aktiven Elektrode (3) aufweist,
**dass** die Zugstange (17) über einen elektrischen Isolierkörper mit der Stirnseite des proximalen Endes (9) der aktiven Elektrode (3) und mit einem Linear-Schrittmotor (19) verbunden ist und
**dass** der Weggeber (20) der aktiven Elektrode (3) auf der Zugstange (17) angeordnet ist und auf der Strecke von der Ausgangsstellung funktioniert, wenn die Spitze (6) der genannten aktiven Elektrode (3) die Körperoberfläche des Patienten berührt und bis die vorgegebene maximale Eintauchtiefe im biologischen Gewebe erreicht ist.

4. Einrichtung nach Anspruch 2,
**dadurch gekennzeichnet,**
**dass** das Antriebssystem (16) der aktiven Elektrode (3) einen Druckluftspeicher (21), ein Getriebe (22), einen elektrisch gesteuerten Verteiler (23) mit einem steuerbaren Ventil (24) und einen Druckluftzylinder (25) mit einem Kolben sowie einen Weggeber (27) aufweist,
**dass** der Kolben mittels einer Stange (26) und über einen elektrischen Isolierkörper mit der Stirnseite des proximalen Endes (9) der aktiven Elektrode (3) verbunden ist und
**dass** der Weggeber (27) der aktiven Elektrode (3) an der Kolbenstange angeordnet ist und auf der Strecke von der Ausgangsstellung wirkt, wenn die Spitze (6) der genannten aktiven Elektrode (3) die Körperoberfläche des Patienten berührt und bis die vorgegebene maximale Eintauchtiefe im biologischen Gewebe erreicht ist.

5. Einrichtung nach Anspruch 1,
**dadurch gekennzeichnet,**
**dass** das System (28) für die Zuführung der leitfähigen Flüssigkeit (A) einen Behälter (10) für die leitfähige Flüssigkeit (A) unter Druck, eine Pumpe (30), einen Druckgeber (31), ein elektrisch gesteuertes Ventil (32) und eine Leitung (34) für die Zuführung der leitfähigen Flüssigkeit (A) in den Innenraum der aktiven Elektrode (3) aufweist.

6. Einrichtung nach Anspruch 1,
**dadurch gekennzeichnet,**
**dass** das Hochfrequenz-Stromversorgungssystem (35) einen Hochfrequenz-Impulsgenerator (36), einen Stromgeber (37) und einen Integrator (38) zur Berechnung der Strommenge, die an die Spitze (6) der Elektrode (3) angelegt ist, einen Stromoberwellenanalysator (39) zur Kontrolle des Vorhandenseins der dritten Oberwelle und der Fundamentalschwingung und einen Spektralanalysator (40) aufweist,
**dass** ein Analysegerät eines optischen Plasmastrahlungsspektrums zur Kontrolle der Plasmagastemperatur und des Plasmabetriebs vorgesehen ist, wobei für die Analyse ein Intensitätsverhältnis der Linien der angeregten Natrium- oder Wasserstoffatome im Strahlungsspektrum verwendet ist und das Analysegerät dabei mit einem Lichtleiter (41) ausgerüstet ist, wobei ein Endstück (42) des Lichtleiters (41) im proximalen Ende (9) der aktiven Elektrode (3) in der Nähe seines Innenkanals (5) sitzt.

7. Einrichtung nach Anspruch 6,

**dadurch gekennzeichnet,**

**dass** sie als Hochfrequenz-Impulsgenerator (36) einen HF-Generator aufweist, welcher die Erzeugung von elektrischem Strom mit einer Hauptträgerfrequenz im Frequenzbereich von 0,10 MHz bis 20,00 MHz mit der Möglichkeit einer Modulationsparameterregelung sicherstellt und

**dass** der HF-Generator

- ein Impulspausenverhältnis im Bereich von 1 bis 10,
- eine Impulsfolgefrequenz im Bereich von 1 kHz bis 20 kHz und
- eine Spannungsimpulshöhe bei fehlender Belastung im Bereich von 100 V bis 400 V aufweist.

8.  Einrichtung nach Anspruch 6,
    **dadurch gekennzeichnet,**
    **dass** das Analysegerät des optischen Plasmastrahlungsspektrums einen V-Verzweiger (43) und lichtempfindliche Sensoren (46, 48) aufweist,
    **dass** der V-Verzweiger (43) optisch mit dem genannten Lichtleiter (41) verbunden ist und die Aufteilung der Lichtemission des Plasmas in zwei optische Kanäle (44, 45) sicherstellt,
    **dass** der lichtempfindliche Sensor (46) mit einem Lichtfilter (47) zur Aussonderung einer Na-Emissionslinie von 337,1 nm oder einer Wasserstoffatomlinie $H_\beta$ von 486,1 nm versehen ist und
    **dass** der andere lichtempfindliche Sensor (48) mit einem Lichtfilter (49) zur Aussonderung einer Na-Emissionslinie von 588,9 nm oder einer Wasserstofflinie $H_\alpha$ von 656,3 nm versehen ist.

9.  Einrichtung nach Anspruch 1,
    **dadurch gekennzeichnet,**
    **dass** der Mikrorechner der automatischen Steuerungseinheit (50) für die Erstellung und die zugriffsbereite Speicherung von wenigstens folgenden Datenbanken angepasst ist:

    - der Intensität des Stroms, welcher die Funktion des Stromgebungssystems im Plasma-Erzeugungsbetrieb je nach den Kennwerten der Impulseinwirkung sicherstellt,
    - der Plasmatemperatur je nach dem Verhältnis der Strahlenintensitäten der Na- und Wasserstofflinien beim Übergang auf höhere Energieebenen,
    - dem Gesamtwert der durch das Plasma durchlaufenden Ladung,
    - der Elektrodenverfahrgeschwindigkeit je nach der Größe des durch das Plasma fließenden Stroms,
    - der Menge der leitfähigen Flüssigkeit, welche für die Bildung des Pufferbereichs (54) je nach dem Durchmesser der aktiven Elektrode (3), ihrer Eintauchtiefe und der Tiefe des Behandlungsgebiets erforderlich ist und
    - der Zuführungsgeschwindigkeit der leitfähigen Flüssigkeit (A) je nach der Menge der leitfähigen Flüssigkeit (A) im Pufferbereich (54) und der Bewegungsgeschwindigkeit der Elektrode (3).

10. Einrichtung nach Anspruch 9,
    **dadurch gekennzeichnet,**
    **dass** der Mikrorechner für die Erstellung von Datenbanken der Geschwindigkeit $C_{equtv}$ der Erzeugung von Wasserstoffperoxid im Pufferbereich (54) je nach der elektrischen Strommenge q angepasst ist, welche durch das Plasma durchgelaufen ist und
    **dass** die Geschwindigkeit $C_{equtv}$ der Wasserstoffperoxiderzeugung in folgender Weise ermittelbar ist:

$$C_{equtv} = F\left[1 - \exp\left(-\frac{G_0 kq}{V}\right)\right] / k$$

,

wobei
q für die Elektrizitätsmenge in Faraday;
$G_0$ für die Menge von Grammäquivalenten von Wasserstoffperoxid, welche ursprünglich pro Faraday der durchgelaufenen Elektrizität (Anfangsstromausbeute) gebildet ist,
V für die Menge der leitfähigen Flüssigkeit (A) im Pufferbereich (54),
k für die Geschwindigkeitszahl der Wasserstoffperoxid-Zersetzungsreaktion und
F für die Erfahrungszahl, welche von den Kennwerten der leitfähigen Flüssigkeit (A) abhängt,

stehen.

11. Einrichtung nach Anspruch 1,
    **dadurch gekennzeichnet**,
    das als leitfähige Flüssigkeit eine 0,9%ige physiologische Kochsalzlösung eingesetzt ist.

**Fig. 1**

**Fig. 2**

**Fig. 3**

Europäisches
Patentamt

European
Patent Office

Office européen
des brevets

# EUROPÄISCHER RECHERCHENBERICHT

**Nummer der Anmeldung**

EP 11 00 4753

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/054366 A1 (DAVISON PAUL O [US] ET AL) 18. März 2004 (2004-03-18) * Zusammenfassung; Abbildungen 1,2,18,20 * * Absätze [0012], [0084], [0092], [0096], [0099], [0155], [0162] * ----- | 1-11 | INV. A61N1/05 A61N1/44 |
| X,D | US 2003/028189 A1 (WOLOSZKO JEAN [US] ET AL) 6. Februar 2003 (2003-02-06) * das ganze Dokument * ----- | 1-11 | |
| A | WO 2009/146432 A1 (UNIV COLORADO STATE RES FOUND [US]; KOO IL-GYO [US]; MOORE CAMERON A []) 3. Dezember 2009 (2009-12-03) * das ganze Dokument * ----- | 1-11 | |
| A | WO 2006/116252 A2 (UNIV DREXEL [US]; GUTSOL ALEXANDER [US]; FRIDMAN ALEXANDER [US]; FRIED) 2. November 2006 (2006-11-02) * das ganze Dokument * ----- | 1-11 | |
| A | US 6 413 255 B1 (STERN ROGER A [US]) 2. Juli 2002 (2002-07-02) * das ganze Dokument * ----- | 1-11 | **RECHERCHIERTE SACHGEBIETE (IPC)** A61N |
| A | US 2004/111087 A1 (STERN ROGER A [US] ET AL STERN ROGER [US] ET AL) 10. Juni 2004 (2004-06-10) * das ganze Dokument * ----- | 1-11 | |
| A | US 2003/204329 A1 (MARCHITTO KOVIN S [US] ET AL) 30. Oktober 2003 (2003-10-30) * das ganze Dokument * ----- | 1-11 | |
| A | US 2003/139731 A1 (MARCHITTO KEVIN [US] ET AL) 24. Juli 2003 (2003-07-24) * das ganze Dokument * ----- -/-- | 1-11 | |

Der vorliegende Recherchenbericht wurde für alle Patentansprüche erstellt

| Recherchenort | Abschlußdatum der Recherche | Prüfer |
|---|---|---|
| München | 16. September 2011 | Pereda Cubián, David |

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 11 00 4753

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 6 086 585 A (HOVDA DAVID C [US] ET AL) 11. Juli 2000 (2000-07-11) * das ganze Dokument * ----- | 1-11 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 16. September 2011 | Pereda Cubián, David |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 00 4753

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-09-2011

| Im Recherchenbericht angeführtes Patentdokument | Datum der Veröffentlichung | Mitglied(er) der Patentfamilie | Datum der Veröffentlichung |
|---|---|---|---|
| US 2004054366 A1 | 18-03-2004 | US 2008021447 A1 | 24-01-2008 |
| US 2003028189 A1 | 06-02-2003 | AU 2003248766 A1 | 19-01-2004 |
| | | EP 1571969 A2 | 14-09-2005 |
| | | WO 2004002293 A2 | 08-01-2004 |
| WO 2009146432 A1 | 03-12-2009 | EP 2297377 A1 | 23-03-2011 |
| | | JP 2011522381 A | 28-07-2011 |
| | | US 2011139751 A1 | 16-06-2011 |
| | | WO 2010138102 A1 | 02-12-2010 |
| | | WO 2010138103 A1 | 02-12-2010 |
| | | WO 2010138104 A1 | 02-12-2010 |
| | | WO 2010138105 A1 | 02-12-2010 |
| WO 2006116252 A2 | 02-11-2006 | AU 2006239843 A1 | 02-11-2006 |
| | | CA 2605650 A1 | 02-11-2006 |
| | | EP 1876986 A2 | 16-01-2008 |
| | | JP 2008539007 A | 13-11-2008 |
| | | US 2010145253 A1 | 10-06-2010 |
| US 6413255 B1 | 02-07-2002 | AT 298536 T | 15-07-2005 |
| | | AU 779100 B2 | 06-01-2005 |
| | | CA 2364098 A1 | 14-09-2000 |
| | | DE 60021063 D1 | 04-08-2005 |
| | | DE 60021063 T2 | 11-05-2006 |
| | | EP 1158919 A1 | 05-12-2001 |
| | | ES 2240078 T3 | 16-10-2005 |
| | | JP 4102031 B2 | 18-06-2008 |
| | | JP 2002537939 A | 12-11-2002 |
| | | WO 0053113 A1 | 14-09-2000 |
| US 2004111087 A1 | 10-06-2004 | AT 411778 T | 15-11-2008 |
| | | AU 2003207858 A1 | 02-09-2003 |
| | | BR 0307392 A | 09-11-2004 |
| | | CA 2474421 A1 | 14-08-2003 |
| | | CN 1627923 A | 15-06-2005 |
| | | EP 1471845 A1 | 03-11-2004 |
| | | EP 1808145 A2 | 18-07-2007 |
| | | ES 2314180 T3 | 16-03-2009 |
| | | JP 2005516666 A | 09-06-2005 |
| | | WO 03065916 A1 | 14-08-2003 |
| | | US 2009287207 A1 | 19-11-2009 |
| | | US 2007010811 A1 | 11-01-2007 |
| US 2003204329 A1 | 30-10-2003 | US 2009143715 A1 | 04-06-2009 |

Für nähere Einzelheiten zu diesem Anhang : siehe Amtsblatt des Europäischen Patentamts, Nr.12/82

EPO FORM P0461

**ANHANG ZUM EUROPÄISCHEN RECHERCHENBERICHT
ÜBER DIE EUROPÄISCHE PATENTANMELDUNG NR.**

EP 11 00 4753

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-09-2011

| Im Recherchenbericht angeführtes Patentdokument | Datum der Veröffentlichung | Mitglied(er) der Patentfamilie | Datum der Veröffentlichung |
|---|---|---|---|
| US 2003139731 A1 | 24-07-2003 | KEINE | |
| US 6086585 A | 11-07-2000 | US 2006253117 A1<br>US 7131969 B1 | 09-11-2006<br>07-11-2006 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- RU 2202382 C2
- RU 2383365 C1

- US 20030028189 A1

**In der Beschreibung aufgeführte Nicht-Patentliteratur**

- **B. T. GLUKHEN'KY ; V. V. DELEKTORSKY ; R. F. FYODOROVSKAYA.** *Pustulöse Hauterkrankungen,* 1983

- **MARC R.AVRAM ; SANDY TSAO.** Color Atlas of Cosmetic Dermatology. Mc Grow Hill Medical, 2006, 295
- **A. V. SHAPIRO.** *Antibiotika und ihre Anwendung bei eitrigen Entzündungen,* 1999